(19) Europäisches Patentamt
European Patent Office
Office européen des brevets

(11) EP 0 684 948 B1

# (12) EUROPÄISCHE PATENTSCHRIFT

(45) Veröffentlichungstag und Bekanntmachung des Hinweises auf die Patenterteilung:
**22.10.1997 Patentblatt 1997/43**

(21) Anmeldenummer: **94908251.5**

(22) Anmeldetag: **11.02.1994**

(51) Int Cl.[6]: **C07D 487/22**, A61K 31/40, A61K 49/00, C07F 9/6561, A61K 31/675 // (C07D487/22, 257:00, 209:00, 209:00, 209:00, 209:00)

(86) Internationale Anmeldenummer:
**PCT/DE94/00159**

(87) Internationale Veröffentlichungsnummer:
**WO 94/19352 (01.09.1994 Gazette 1994/20)**

## (54) MESO-TETRAPHENYLPORPHYRIN-KOMPLEXVERBINDUNGEN, VERFAHREN ZU IHRER HERSTELLUNG UND DIESE ENTHALTENDE PHARMAZEUTISCHE MITTEL

MESO-TETRAPHENYL PORPHYRIN COMPLEX COMPOUNDS, PROCESS FOR PREPARING THE SAME AND PHARMACEUTICALS CONTAINING THE SAME

COMPOSES COMPLEXES DE MESO-TETRAPHENYLPORPHYRINE, LEUR PROCEDE DE PREPARATION ET AGENTS PHARMACEUTIQUES LES CONTENANT

(84) Benannte Vertragsstaaten:
**AT BE CH DE DK ES FR GB GR IE IT LI LU MC NL PT SE**

(30) Priorität: **17.02.1993 DE 4305523**

(43) Veröffentlichungstag der Anmeldung:
**06.12.1995 Patentblatt 1995/49**

(73) Patentinhaber: **INSTITUT FÜR DIAGNOSTIKFORSCHUNG GmbH AN DER FREIEN UNIVERSITÄT BERLIN**
**14050 Berlin (DE)**

(72) Erfinder:
- **MAIER, Franz, Karl**
  **D-13467 Berlin (DE)**
- **EBERT, Wolfgang**
  **D-12203 Berlin (DE)**
- **LEE-VAUPEL, Mary**
  **D-10711 Berlin (DE)**
- **GRIES, Heinz**
  **D-10717 Berlin (DE)**
- **CONRAD, Jürgen**
  **D-12163 Berlin (DE)**

(56) Entgegenhaltungen:
**EP-A- 0 336 879**

- **INORGANIC CHEMISTRY Bd. 31, Nr. 26 , 1992 , EASTON US Seiten 5433 - 5438 E. KEIMAN ET AL. 'Catalytic Antibodies. Circular dichroism and UV-Vis studies of antibody-matalloporphyrin interactions'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 684 948 B1

Printed by Jouve, 75001 PARIS (FR)

## Beschreibung

Die Erfindung betrifft den in den Patentansprüchen gekennzeichneten Gegenstand, das heißt neue meso-Tetraphenylporphyrin-Komplexverbindungen, diese Verbindungen enthaltende neue pharmazeutische Mittel, ihre Verwendung in Diagnostik und Therapie sowie Verfahren zur Herstellung dieser Verbindungen und Mittel.

Die Anwendung von Komplexbildnern oder Komplexen bzw. deren Salzen in der Medizin ist seit langem bekannt. Als Beispiele seien genannt: Komplexbildner als Stabilisatoren pharmazeutischer Präparate, Komplexe und deren Salze als Hilfsmittel zur Verabreichung schlecht löslicher Ionen (z.B. Eisen), Komplexbildner und Komplexe (bevorzugt Calcium- oder Zink-), gegebenenfalls als Salze mit anorganischen und/oder organischen Basen, als Antidots zur Entgiftung bei versehentlicher Inkorporation von Schwermetallen oder deren radioaktiven Isotopen und Komplexbildner als Hilfsmittel in der Nuklearmedizin unter Verwendung radioaktiver Isotope wie $^{99m}Tc$ für die Szintigraphie sind bekannt. In den Patentschriften EP 71564, EP 130934 und DE-OS 3401052 sind Komplexe und Komplexsalze als Diagnostika, vorwiegend als NMR-Diagnostika, vorgestellt worden. Diese Komplexe und Komplexsalze sind recht gut verträglich und gewährleisten eine weitestgehende vollständige Ausscheidung der Ionen. Noch nicht optimiert sind deren die Wirksamkeit eines NMR-Kontrastmittels bestimmenden Parameter, von denen genannt werden:
eine günstige Relaxivität, so daß das Kontrastmittel in möglichst geringen Konzentrationen die Relaxationszeit der Protonen im Gewebewasser und anderer Kerne wie P, F und Na in-vivo erniedrigt und damit beispielsweise die Lokalisation von Tumoren durch Erhöhung der Signalintensität des mit Hilfe des Kernspintomographen erhaltenen Bildes ermöglicht; eine möglichst selektive Anreicherung und/oder Retention des Kontrastmittels im Zielorgan; ausreichende Wasserlöslichkeit; geringe Toxizität; gute Verträglichkeit; gute chemische und biochemische Stabilität.

Die Komplexe und Komplexsalze der genannten Patentschriften sind mit dem Nachteil behaftet, daß sie sich nur relativ unspezifisch im Extrazellulärraum verteilen und daher nicht immer eine Erkennung pathologisch veränderter Gewebe ermöglichen. Es besteht daher ein Bedarf nach vor allem selektiv-bindenden, tumorspezifischen Verbindungen. Bezüglich ihrer Verträglichkeit sollten sie einen möglichst großen Sicherheitsabstand aufweisen. Als Maß hierfür kann das Produkt aus Toxizität und Relaxivität betrachtet werden.

E. Keiman et al. beschreiben in Inorganic Chemistry, Bd. 31, Nr. 26, 1992, 5433-5438 CD- und UV-Messungen zum Studium der Interaktionen zwischen Metallporphyrinen und monoklonalen Antikörpern (MABs), die in der Absicht durchgeführt wurden, die Faktoren, die die katalytische und andere Aktivitäten der MAB-Metallporphyrin-Komplexe (z. B. reversible Sauerstoffbindung) bestimmen, besser zu verstehen. Es ist dieser Literaturstelle kein Hinweis auf die Verwendung dieser Verbindungen für die NMR-Diagnostik, RadioDiagnostik oder Radio-Therapie zu entnehmen.

Es ist nun seit einigen Jahren bekannt, daß sich Porphyrinderivate selektiv in menschlichen und tierischen Tumoren anreichern können (D. Kessel und T.-H. Chou, Cancer Res 43, pp 1994-1999, 1983, P. Hambright, Bioinorg. Chem. 5, pp 87-92, 1975; R. Lipson et al., Cancer 20, p 2250-2257, 1967; D. Sanderson et al., Cancer 30, pp. 1368-1372, 1972). Erste Ansätze, diese Verbindungsklasse auch als Diagnostika einzusetzen sind ebenfalls beschrieben worden (J. Winkelmann et al., Cancer Research 27, pp. 2060-2064, 1967; Europäische Patentanmeldung Publikations Nr. 133603; N.J. Patronas et al., Cancer Treatment Reports 70, pp. 391-395, (1986).

Die bisher beschriebenen Verbindungen sind jedoch weit davon entfernt die oben genannten Kriterien zufriedenstellend zu erfüllen; besondere Aufmerksamkeit verlangt immer noch ihre mangelnde Anreicherung in den Zielorganen. Eine Verbesserung dieser Eigenschaft sollte gleichzeitig die bestehenden Probleme mit der Toxizität und Verträglichkeit der vorbekannten Verbindungen reduzieren helfen.

In der Patentanmeldung EP 0336879 werden substituierte meso-Tetraphenylporphyrin-Komplexverbindungen zur Anwendung in der Diagnostik und Therapie beschrieben. Zwar zeigen diese Verbindungen ein gutes Anreicherungsverhalten in verschiedenen Zielorganen, jedoch weisen die beschriebenen Verbindungen bei der Verwendung als NMR-Diagnostika ein noch nicht völlig befriedigendes Verhältnis zwischen der für eine optimale Bildgebung erforderlichen Dosis und der letalen Dosis auf.

Es besteht daher weiterhin für vielfältige Zwecke ein Bedarf an stabilen, gut löslichen, aber auch besser verträglichen und selektiver bindenden Komplexverbindungen, die für die Diagnostik und/oder auch Therapie beispielsweise von Tumoren geeignet sind. Der Erfindung liegt somit die Aufgabe zugrunde, diese Verbindungen und pharmazeutischen Mittel zur Verfügung zu stellen, sowie ein Verfahren zu ihrer Herstellung zu schaffen.

Diese Aufgabe wird durch die Erfindung gelöst.

Es wurde gefunden, daß sich die erfindungsgemäßen Porphyrin-Komplexverbindungen, bestehend aus neuen meso-Tetraphenylporphyrinliganden und Ionen eines Elements der Ordnungszahlen 21-32, 38, 39, 42-51 oder 57-83 sowie gegebenenfalls einem oder mehreren Kation(en) anorganischer und/oder organischer Basen, Aminosäuren oder Aminosäureamide, überraschenderweise hervorragend zur Herstellung von NMR-Diagnostika, Radio-Diagnostika und Radio-Therapeutika eignen.

Die erfindungsgemäßen Porphyrin-Komplexverbindungen bestehen aus einem meso-Tetraphenylporphyrinliganden der allgemeinen Formel I

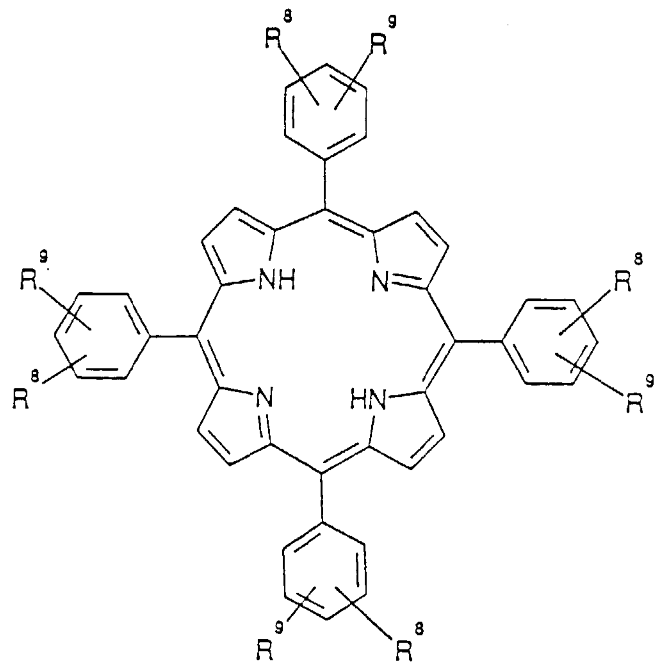

(I),

worin

$R^8$ für einen Rest $-(O)_s-(CH_2)_k-X-CH_2-Y-C(=O)-Z$ mit

s in der Bedeutung der Ziffern 0 oder 1,
k in der Bedeutung der Ziffern 0, 1, 2 oder 3,
X in der Bedeutung eines Sauerstoffatoms, einer direkten Bindung oder einer $-NR^{10}$-Gruppe wobei
$R^{10}$ für eine $C_1$-$C_4$-Acyl-, $C_1$-$C_{10}$-alkylsulfonyl-, Benzolsulfonyl-, $C_1$-$C_4$-alkylphenylensulfonyl-, Carboxy-$C_1$-$C_6$-alkyl oder Carboxy-$C_1$-$C_5$-acylgruppe,
Y in der Bedeutung einer direkten Bindung oder einer-CHOH-Gruppe und
Z in der Bedeutung einer-OH-oder $-N(R^{11})-R^{12}$ Gruppe steht, wobei
$R^{11}$ und $R^{12}$ unabhängig voneinander für Wasserstoff oder für einen gegebenenfalls durch 1 - 6 Hydroxygruppen substituierten gerad- oder verzweigtkettigen oder cyclischen, gesättigten oder ungesättigten Kohlenwasserstoffrest mit bis zu 16 C-Atomen stehen,
mit der Maßgabe, daß keine direkten O-O oder O-N-Bindungen gestattet sein sollen,

$R^9$ für ein Wasserstoff-, Fluor-, Chlor-, Brom-, Jodatom, einen geradkettigen oder verzweigten $C_1$-$C_4$-Alkyrest oder für einen der für $R^8$ angegebenen Substituenten stehen, und einem Ion eines Elements der Ordnungszahlen 21 - 32, 38, 39, 42 - 51 oder 58 - 83 sowie gegebenenfalls einem oder mehreren physiologisch unbedenklichen Kation (en) anorganischer und/oder organischer Basen, Aminosäuren oder Aminosäureamiden mit Ausnahme der Eisen-, Nickel-, Mangan- und Zinn Komplexe des meso-5,10,15,20-Tetrakis-[4-carboxylatomethoxy)-phenyl]-porphyrins bestehen.

Sind die erfindungsgemäßen Mittel zur Anwendung in der NMR-Diagnostik bestimmt, so müssen paramagnetische Metallionen im Komplex vorhanden sein. Dies sind insbesondere die zwei- und dreiwertigen Ionen der Elemente der Ordnungszahlen 21-29, 42, 44 und 58-70. Geeignete Ionen sind beispielsweise das Chrom(III)-, Mangan(III)-, Eisen (III)-, Kobalt(II)-, Kobalt(III)-, Nickel(II)-, Kupfer(II)-, Praseodym(III), Neodym(III)-, Samarium(III)-, Gadolinium(III), Terbium(III)-, Dysprosium(III)-, Holmium(III)-, Erbium(III)- und Ytterbium(III)-ion. Besonders bevorzugt ist das Mangan(III)-ion wegen der hohen Stabilität und des hohen magnetischen Moments seiner Porphyrinkomplexe.
Für die Radiodiagnostik und Radiotherapie sind Komplexe geeignet, die als Zentralatom ein Radioisotop der Elemente 27, 29 - 32, 38, 39, 42 - 51, 62, 64, 70, 75, 77, 81, 82 oder 83 enthalten. Besonders geeignet sind zum Beispiel Radioisotope der Elemente Kupfer, Kobalt, Gallium, Germanium, Yttrium, Strontium, Technetium, Indium, Ytterbium, Gadolinium, Samarium, Thallium und Iridium.

Sofern eines der vom Porphyrinliganden komplexierten Ionen in einer höheren Oxidationsstufe als +2 vorliegt,

wird (werden) die überschüssigen Ladung(en) durch Anionen von organischen oder anorganischen Säuren, bevorzugt durch Acetat- und Chlorid-, gegebenenfalls auch durch Oxid- und Nitridanionen ausgeglichen.

Überraschenderweise zeigen die erfindungsgemäßen Komplexe gegenüber den bisher bekannten, strukturell ähnlichen Verbindungen eine deutlich höhere Relaxivität. Da die Relaxivität einen Hinweis auf die Kontrastmittelwirksamkeit einer Verbindung gibt, gelingt bei Verwendung der erfindungsgemäßen Komplexe im Bereich der NMR-Diagnostik eine vergleichbare, positive Signalbeeinflussung schon bei einer niedrigeren Dosis. Dadurch vergrößert sich der Sicherheitsabstand signifikant, für den als Richtwert das Produkt aus Relaxivität und Verträglichkeit angesehen werden kann.

Die erfindungsgemäßen Verbindungen erweisen sich im direkten Vergleich auch den in der Patentanmeldung EP 0 336 879 beschriebenen Verbindungen bezüglich ihrer Wirksamkeit und ihrer Verträglichkeit als überlegen (Siehe Tabelle Beispiel 17).

Mit Hilfe der erfindungsgemäßen Komplexverbindungen können überraschenderweise nicht nur Tumorgewebe und einzelne Organe wie zum Beispiel Leber, Nieren und Lymphknoten, sondern auch Blutgefäße ohne Anwendung spezieller Puls-Sequenzen in-vivo dargestellt werden, womit sie unter anderem als Perfusionsagentien Verwendung finden können.

Als Alkylsubstituenten $R^{11}$ und $R^{12}$ kommen gesättigte, ungesättigte gerad- oder verzweigkettige oder cyclische Kohlenwasserstoffe mit bis zu 16 C-Atomen, vorzugsweise gesättigte Kohlenwasserstoffe mit 1 bis 10 C-Atomen, insbesondere gesättigte Kohlenwasserstoffatome mit 1 bis 7 C-Atomen in Betracht, die gegebenenfalls durch 1 bis 5 Hydroxy-Gruppen substitutiert sind.

Als gegebenenfalls substituierte Alkylgruppen seien beispielsweise die Methyl-, Ethyl-, 2-Hydroxyethyl-, 2-Hydroxy-1-(hydroxymethyl)-ethyl-, 1-(Hydroxymethyl)-ethyl-, Propyl-, Isopropyl, 2-Hydroxypropyl-, 3-Hydroxypropyl-, 2,3-Dihydroxypropyl-, Butyl-, Isobutyl-, Isobutenyl-, 2-Hydroxybutyl-, 3-Hydroxy-butyl-, 4-Hydroxybutyl-, 2,-3- und 4-Hydroxy-2-methylbutyl-, 2- und 3-Hydroxy-isobutyl-, 2,3,4-Trihydroxybutyl-, 1,2,4-Trihydroxybutyl-, Pentyl-, Cyclopentyl-, Cyclohexyl-, 2,3,4,5,6-Pentahydroxyhexylgruppe genannt.

Für $R^{10}$ seien beispielsweise genannt: der Formyl-, Acetyl-, Propionyl-, n-Butyryl-, i-Butyryl-, Methansulfonyl-, Ethansulfonyl-, Propansulfonyl-, Butansulfonyl-, Pentansulfonyl-, Hexansulfonyl-, Heptansulfonyl-, Oktansulfonyl-, Nonansulfonyl-, Decansulfonyl-, Benzolsulfonyl-, p-Toluolsulfonyl-, 4-Ethyl-phenylsulfonyl-, Carboxyhexyl-, Carboxypentyl-, Carboxybutyl-, Carboxypropyl-, Carboxyethyl, Carboxymethyl-, 5-Carboxyvaleroyl-, 4-Carboxybutyryl-, 3-Carboxypropionyl-, Carboxyacetyl-, Carboxycarbonylrest.

In $R^8$ und/oder $R^9$ gegebenenfalls vorhandene acide Wasserstoffatome können gegebenenfalls ganz oder teilweise durch Kationen anorganischer und/oder organischer Basen oder Aminosäuren oder Aminosäureamide ersetzt sein. Geeignete anorganische Kationen sind beispielsweise das Lithiumion, das Kaliumion, das Calciumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die Herstellung der erfindungsgemäßen Porphyrin-Komplexverbindungen der Formel I erfolgt dadurch, daß man Porphyrine der allgemeinen Formel II

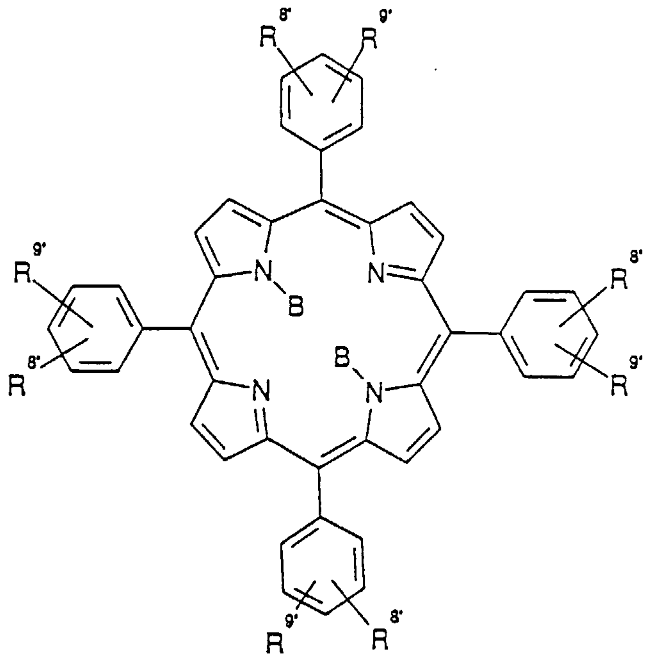

(II),

worin

B für ein Wasserstoffatom oder ein Metallionenäquivalent eines Elements der Ordnungszahlen 21-32, 38, 39, 42-51 oder 58-83,

$R^{8'}$ für einen Rest $-(O)_s-(CH_2)_k-X'-CH_2-Y'-C(=O)-Z'$ mit X' und Y' in der für X und Y angegebenen Bedeutung, wobei in X und Y gegebenenfalls enthaltene funktionelle Gruppen jedoch gegebenenfalls geschützt sind und Z' in der Bedeutung einer Fluchtgruppe,

$R^{9'}$ für $R^9$ oder für einen der für $R^{8'}$ angegebenen Substituenten stehen, verseift oder mit einem Amin der allgemeinen Formel III

$$H\,N\,R^{11'}\,R^{12'} \qquad \text{(III)},$$

worin
$R^{11'}$ und $R^{12'}$ die für R11 und R12 angegebenen Bedeutung haben, wobei in $R^{11}$ und $R^{12}$ gegebenenfalls enthaltene Hydroxygruppen jedoch gegebenenfalls geschützt sind, umsetzt, gegebenenfalls vorhandene Schutzgruppen abspaltet und in den Fällen wo B für ein Wasserstoffatom steht, mit einem Metalloxid oder Metallsalz eines Elements der Ordnungszahlen 21-32, 38, 39, 42-51 oder 58-83 umsetzt - wobei die Reihenfolge der beiden letztgenannten Umsetzungen vertauscht werden kann - und gewünschtenfalls anschließend in den so erhaltendenen Porphyrinkomplexverbindungen gegebenenfalls noch vorhandene acide Wasserstoffatome durch Kationen anorganischer und/oder organischer Basen, Aminosäuren und Aminosäureamide substituiert.

Funktionelle Gruppen in Vorstufen der erfindungsgemäßen Verbindungen mit den Inkrementen und Resten X, Y, $R^{11}$, $R^{12}$ in der oben genannten Bedeutung können mit Schutzgruppen versehen werden, um z.B. selektive Reaktionen, eine leichtere Aufreinigung oder gute Löslichkeit in organischen Lösungsmitteln zu gewährleisten.

So können in X gegebenenfalls enthaltene Carboxyfunktionen als Ester geschützt werden. Bevorzugt sind Methyl-, Ethyl-, Isopropyl- und tert.-Butylester. Hydroxygruppen, die in Y, $R^{11}$, $R^{12}$ enthalten sein können, können beispielsweise als Ester, Diole oder Acetale geschützt werden. Insbesondere für einwertige Alkohole wird der Acetylrest als Schutzgruppe bevorzugt, zum Schutz von Diolen und gegebenenfalls auch von höherwertigen Alkoholen wird (werden) darüber hinaus (der) Isopropyliden - und (der) Benzylidenrest(e) besonders bevorzugt.
Die Einführung und Abspaltung der Schutzgruppen, gelingt in an sich bekannter Weise (Th. W. Greene, P.G.M. Wuts:

Protective Groups in Organic Synthesis, John Wiley & Sons Inc. (1991) 10, 143, 224).

Z' in der oben genannten Bedeutung als Fluchtgruppen können z.B. für Halogenid-, Alkoholat-, Acyloxy-, Alkyloxycarbonyloxy- Reste stehen.

Sollen durch Abspaltung oder Umwandlung der Reste Z' Carboxy- bzw. Carboxylatfunktionen entstehen, werden für Z' Methoxy-, Ethoxy-, Propoxy- oder tert.-Butoxyreste bevorzugt. Bezüglich der Blockierung und Freisetzung der Carboxy- bzw. Carboxylatfunktionen sei auf die oben zur Handhabung von Schutzgruppen zitierte Literatur verwiesen.

Sollen die Reste Z' als Nucleofug bei der Acylierung von Aminen der allgemeinen Formel III dienen, werden für Z' folgende Reste bevorzugt: der Chlor-, Brom-, Jod-, Methoxy-, Ethoxy-, Propoxy-, Acetoxy-, Methoxycarboxy-, Ethoxycarboxy-, n-Propoxycarboxy-, i-Propoxycarboxy-, i-Butoxycarboxy-, tert.-Butoxycarboxy-, 2-Ethylhexyloxycarboxy-, Benzyloxycarboxy-, 2,5-Dioxopyrrolidin-1-yl-oxy-, Benzotriazol-1-yl-oxy- und der Trimethylsilyloxyrest.

Die Bedingungen zur Darstellung aktivierter Carbonylverbindungen und ihre Umsetzung zu Amiden sind literaturbekannt (R.C. Larock, Comprehensive Organic Transformations, VCH Verlagsgesellschaft mbH Weinheim (1989), S. 963, Houben-Weyl, Methoden der Organischen Chemie, Georg Thieme-Verlag, Stuttgart, Band E5 (1985), 633; Org. Reakt. 12, 157 (1952)). Zur Darstellung der gewünschten Amide seien als geeignete Amine der allgemeinen Formel III beispielsweise genannt:

Dimethylamin, Diethylamin, Di-n-propylamin, Diisopropylamin, Di-n-butylamin, Diisobutylamin, Di-sek.butylamin, N-Methylpropylamin, Dioctylamin, Dicyclohexylamin, N-Ethylcyclohexylamin, Diisopropenylamin, Benzylamin, Anilin, 4-Methoxyanilin, 4-Dimethylaminoanilin, 3,5-Dimethoxyanilin, Morpholin, Pyrrolidin, Piperidin, N-Methylpiperazin, N-Ethylpiperazin, N-(2-Hydroxyethyl)-piperazin, N-(Hydroxymethyl)piperazin, Piperazinoessigsäureisopropylamid, N-Piperazinomethylcarbonyl)-morpholin, N-(Piperazinomethylcarbonyl)-pyrrolidin, 2-(2-Hydroxymethyl)-piperidin, 4-(2-Hydroxyethyl)-piperidin, 2-Hydroxymethylpiperidin, 4-Hydroxymethylpiperidin, 2-Hydroxymethyl-pyrrolidin, 3-Hydroxymethyl-piperidin, 4-Hydroxypiperidin, 3-Hydroxy-pyrrolidin, 4-Piperidon, 3-Pyrrolin, Piperidin-3-carbonsäureamid, Piperidin-4-carbonsäureamid, Piperidin-3-carbonsäurediethylamid, Piperidin-4-carbonsäuredimethylamid, 2,6-Dimethylpiperidin, 2,6-Dimethylmorpholin, N-Acetylpiperazin, N-(2-Hydroxy-propionyl)-piperazi n, N-(3-Hydroxypropionyl)-piperazin, N-(Methoxyacetyl)-piperazin, 4-(N-Acetyl-N-methylamino)-piperidin, Piperidin-4-carbonsäure-(3-oxapentamethylen)-amid, Piperidi n-3-carbonsäure-(3-oxapentamethylen)-amid, N-(N',N'-Dimethylcarbamoyl)-piperazin, Pyrazol in, Imidazolin, Oxazolidin, Thiazolidin, 2,3-Dihydroxypropylamin, N-Methyl-2,3-dihydroxypropylamin, 2-Hydroxy-1-(hydroxymethyl)-ethylamin, N,N-Bis-(2-hydroxyethyl)-amin, N-Methyl-2,3,4,5,6-pentahydroxyhexylamin, 6-Amino-2,2-dimethyl-1,3,-dioxepin-5-ol, 2-Hydroxyethylamin, 2-Amino-1,3-propandiol, Diethanolamin, Ethanolamin.

Die Polyhydroxalkylamine können vorteilhafterweise auch in geschützter Form zur Reaktion eingesetzt werden, zum Beispiel als O-Acylderivate oder als Ketale. Dies gilt besonders dann, wenn diese Derivate bequemer und billiger herstellbar sind als die Polyhydroxyalkylamine selbst. Ein typisches Beispiel ist das 2-Amino-1-(2,2-dimethyl-1,3-dioxolan-4-yl)-ethanol, das Acetonid des 1-Amino-2,3,4-trihydroxybutans, hergestellt nach DE-OS 31 50 917.

Die nachträgliche Entfernung der Schutzgruppen ist problemlos und kann zum Beispiel durch Behandlung mit einem sauren Ionenaustauscher in wäßrig-ethanolischer Lösung erfolgen.

Die Einführung der gewünschten Metalle (z.B. Mn, Fe, Co, Ni, Cu, Zn, Tc, Sm, Eu, Gd, Tl, Bi) in die Porphyrine erfolgt nach literaturbekannten Methoden (The Porphyrins, ed. D. Dolphin, Academic Press, New York 1980, Vol. V, p. 459), wobei im wesentlichen zu nennen sind:

a) die Substitution der pyrrolischen NH's (durch Erwärmen des metallfreien Liganden mit dem entsprechenden Metallsalz, vorzugsweise dem Acetat, gegebenenfalls unter Zusatz von säurepuffernden Mitteln, wie z.B. Natriumacetat, in einem polaren Lösungsmittel) oder

b) die "Umkomplexierung" bei der ein bereits von Liganden komplexiertes Metall (z.B. Zink) durch das gewünschte Metall (z.B. Mangan) verdrängt wird.

Als Lösungsmittel sind vor allem polare Solventien, wie z.B. Methanol, Eisessig, Pyridin, N,N-Dimethylformamid, Dichlormethan, Chloroform und Wasser geeignet.

Zur Darstellung von nuklearmedizinischen Präparaten mit kurzer Halbwertzeit des Zentralatoms empfiehlt es sich in der Regel, das gewünschte Metall im letzten Schritt der Synthese in den Porphyrinliganden einzubauen. Ansonsten ist es meist von Vorteil, gegebenenfalls vorhandene Schutzgruppen am Porphyrinliganden erst nach Einführung des gewünschten Metalls durch - wie oben beschrieben - in der Organischen Chemie übliche Methoden zu entfernen, da die geschützten Verbindungen aufgrund ihrer Löslichkeit in organischer Solventien leicht gereinigt werden können, z. B. durch Ausschütteln, Umkristallisieren oder chromatographische Methoden.

Die Herstellung der meso-Tetraphenylporphyrine erfolgt meist dadurch, daß man durch Rothemund-Reaktion (P. Rothemund, J. Am. Chem. Soc. 57, 2010 (1935); 61, 2912 (1939)) - oder eine Variante dieser Methode (A. D. Adler, F. R. Longo, J.D. Finarelli, J. Goldmacher, J. Assour und J. Korsakoff, J. Org. Chem. 32, 476 (1967); O. Bortolini, M. Ricci, B. Meunier, P. Friant, I. Ascone und J. Goulon, Nouv. J. Chimie 10, 39 (1986); C.L. Hill und M.M. Williamson, J.

Chem. Soc. Chem. Commun. 1228 (1985); P.S. Traylor, D. Dolphin und T.G. Traylor, J. Chem. Soc. Chem. Commun. 279 (1984); J.S. Lindsey, I.C. Schreiman, H.C. Hsu, P.C. Kearney und A. M. Marguerettaz, J. Org. Chem. 52, 827 (1987); A.W. van der Made, E.J.H. Hoppenbrouwer, R.J.M. Nolte und W. Drenth, Recl. Trav. Chim. Pays-Bas 107, 15-16 (1988)) - ein gegebenenfalls geeignet substituiertes Pyrrol mit einem geeignet mono- oder disubstituierten Benzaldehyd zum Makrocyclus umsetzt.

Hierfür besonders bevorzugte Pyrrole sind Pyrrol, 3,4-Dimethylpyrrol und 3,4-Diethylpyrrol (CA 109 (9) : 73206a).

Bevorzugte Benzaldehydderivate sind z.B. 2-Hydroxybenzaldehyd, 3-Hydroxybenzaldehyd, 4-Hydroxybenzaldehyd, 2,3-Dihydroxybenzaldehyd, 3,4-Dihydroxybenzaldehyd, die im Handel erhältlich sind. Es ist in der Regel von Vorteil die Hydroxyfunktion der aufgeführten Benzaldehydderivate vor der Rothemund-Reaktion mit geeigneten Schutzgruppen zu versehen, z.B. mit Methyl-, Acetyl- oder Benzylschutzgruppen, die nach dem Aufbau des Makrocyclus wieder leicht entfernt werden können. (Th. W. Greene, P.G. M. Wats: Protective Groups in Organic Synthesis, John Wiley R. Sons Inc., (1991) 10, 143; Michel Momenteau et al., J. Chem. Soc., Perkin Trans. 1(1983)189).
Besonders bevorzugte Benzaldehydderivate sind 3-Brommethylbenzaldehyd (B.C. Bookser und T.C. Brenice, J. Am. Chem. Soc. 1991, 113, 4208-4218), (2-Formylphenoxy)-essigsäure-i-propylester (CA 84 (11) : 74117z; 80 (15): 82705 z), 2,2'-[(4-Formyl-1,2-phenylen)-bis(oxy)]-bis-essigsäurediethylester (CA 110 (13) : 114844 y), (3-Formylphenyl)-essigsäuremethylester (Baker et al., J. Chem. Soc. 1956, 404, 413), 3-(3-Formylphenyl)-propionsäureethylester (CA 107 (3) : 115500 h), N,N-Bis-[(1-methylethyl)-oxycarbonylmethyl]-3-aminobenzaldehyd (s. Beispiel 8b).

Während Alkylsubstituenten am Pyrrolring des Porphyrins in der Regel vorteilhafterweise durch Verwendung von entsprechend substituiertem Pyrrol für die Rothemundreaktion etabliert werden (s.o.), werden Halogensubstituenten leicht durch Halogenierung des Porphyrins in dessen Pyrrolringe eingeführt (z.B.: S. Onaka et al., Poster 34, 29th International Conference on Coordination Chemistry, Lausanne, Switzerland (1992)).

Folgende Porphyrine werden als Ausgangsmaterial bzw. als Zwischenstufe zur Herstellung der erfindungsgemäßen Porphyrin-Komplexverbindungen besonders bevorzugt: 5,10,15,20-Tetrakis-(2-hydroxyphenyl)-porphyrin, 5,10,15,20-Tetrakis-(3-hydroxyphenyl)-porphyrin, 5,10,15,20-Tetrakis-(4-hydroxyphenyl)-porphyrin (R. Bonnett et al., Photobiochemistry and Biophysics, Suppl. (1987) 45-56), 5,10,15,20-Tetrakis-(3-acetylaminophenyl)-porphyrin (Beispiel 5a), 5,10,15,20-Tetrakis-(3-brom-methylphenyl)-porphyrin, 5,10,15,20-Tetrakis-(3-aminomethylphenyl)-porphyrin, 5,10,15,20-Tetrakis-[3-(p-toluolsulfonamidomethyl)-phenyl]-porphyrin (T.C. Bruice et al., J. Am. Chem. Soc. 1991, 113, 4208), 5,10,15,20-Tetrakis-(3-cyanomethylphenyl)-porphyrin (T.C. Bruice et al., J. Org. Chem. 56 (11), 3470 (1991)).
So gelingt die Veretherung insbesondere phenolischer OH-Gruppen in Tetraphenylporphyrinen mit in der Organischen Chemie gängigen Elektrophilen wie z.B. geeignet substituierten Alkylchloriden - bromiden, - iodiden, - tosylaten oder Epoxiden (Houben-Weyl, Methoden der organischen Chemie, Band VI/3, Georg Thieme Verlag Stuttgart (1965), 7). Als Lösungsmittel können sowohl unpolare Solventien wie z.B. Toluol und Benzol insbesondere bei Verwendung von Phasentransferkatalysatoren (z.B. Triethylbenzylammoniumbromid), polare aprotische Solventien z.B. Diethylether, Tetrahydrofuran, Dioxan, N,N-Dimethylformamid und Dimethylsulfoxid als auch protische Solventien wie z.B. Wasser, Methanol, Ethanol und als Hilfsbasen z.B. tertiäre Amine wie Triethylamin oder anorganische Basen z.B. Lithium-, Natrium- oder Kaliumcarbonat, -hydrogencarbonat, -hydroxid zur Anwendung kommen. Primäre Carbonsäure- bzw. Sulfonsäureamidfunktionen in den Tetraphenylporphyrinsubstituenten können mit geeigneten Alkylierungsmitteln in sekundäre Carbonsäure- bzw. Sulfonsäureamide umgesetzt werden (Houben-Weyl, Methoden der organischen Chemie, Band VIII, Georg Thieme Verlag Stuttgart (1952), 709, Band IX (1955), 405). Auch hierfür können die oben beispielhaft aufgeführten Lösungsmittel und Hilfsbasen verwendet werden.
Nucleofug-substituierte, d.h. z.B. durch Chlor, Brom, Jod, Toluolsulfonat substituierte Alkylseitenketten von Tetraphenylporphyrinen lassen sich z.B. in an sich bekannter Weise in nitril- und diese in carboxysubstituierte Seitenketten überführen (Houben-Weyl, Methoden der organischen Chemie, Band VIII, Georg Thieme Verlag Stuttgart (1952) 265).

Die Neutralisation der gegebenenfalls als Säuren anfallenden Komplexe erfolgt gewünschtenfalls mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl-und N,N-Dimethylglucamin, sowie basischer Aminosäuren, wie zum Beispiel Lysin, Arginin und Ornithin oder von Amiden ursprünglich neutraler oder saurer Aminosäuren.
Zur Herstellung der neutralen Komplexverbindungen kann man beispielsweise den als (Oligo-) Säuren isolierten Metalloporphyrinen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten.
Im Falle der Verwendung von Radioisotope enthaltenden Komplexverbindungen kann deren Herstellung nach den in

"Radiotracers for Medical Applications", Volume 1CRC-Press, Boca Raton, Florida beschriebenen Methoden vorgenommen werden.
Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Komplexbildnern (wie zum Beispiel Diethylentriaminpentasessigsäure) oder, falls erforderlich, Elektrolyte wie zum Beispiel Natriumchlorid oder falls erforderlich, Antioxidantien wie zum Beispiel Ascorbinsäure. Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff (en) (zum Beispiel Methylcellulose, Lactose, Mannit) und/oder Tensid(en) (zum Beispiel Lecithine, Tween®, Myrj®, Taurocholat) und/oder Aromastoff(en) zur Geschmackskorrektur (zum Beispiel ätherischen Ölen) gemischt. Sie können auch in Form von Kapseln bei oraler Applikation oder als Liposomen angewendet werden.
Es muß besondere Sorgfalt darauf verwendet werden, die Komplexierung so vorzunehmen, daß die erfindungsgemäßen Metalloporphyrine und ihre Lösungen praktisch frei sind von nicht komplexierten toxisch wirkenden Metallionen. Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zu Herstellung der Komplexverbindungen und ihrer Salze. Als letzte Sicherheit bleibt eine Reinigung des isolierten Komplexsalzes. Um unerwünschte Photoreaktionen der Porphyrine zu vermeiden, sollten die erfindungsgemäßen Verbindungen und Mittel möglichst unter Lichtausschluß gelagert und gehandhabt werden.
Die erfindungsgemäßen pharmazeutischen Mittel enthalten vorzugsweise 20 µMol/L bis 200 mMol/L des Komplexsalzes und werden in der Regel in Mengen von 0,01 µMol bis 2,0 mMol/kg Körpergewicht dosiert, wobei die bevorzugte Dosis für die NMR-Diagnostik 0,01 - 0,50 mMol/kg Körpergewicht beträgt. Die erfindungsgemäßen Komplexverbindungen sind zur enteralen und parenteralen Applikation bestimmt und kommen zur Anwendung

1. für die NMR-Diagnostik in Form ihrer Komplexe mit den Ionen der Elemente mit den Ordnungszahlen 21-29, 42, 44 und 58-70;
2. für die Radiodiagnostik und Radiotherapie in Form ihrer Komplexe mit den Radioisotopen der Elemente mit den Ordnungszahlen 27, 29-32, 38-39, 42-51, 62, 64, 70, 75, 77, 82 und 83.

Die Wasserlöslichkeit der erfindungsgemäßen Mittel erlaubt es, konzentrierte Lösungen herzustellen, damit die Volumenbelastung des Kreislaufs in vertretbaren Grenzen zu halten und die Verdünnung durch Körperflüssigkeit auszugleichen. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.
Ferner können die erfindungsgemäßen Komplexverbindungen vorteilhaft als Suszeptibilitäts-Reagenzien und als shift-Reagenzien für die in-vivo NMR-Spektroskopie verwendet werden.
Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida beschrieben. Eine weitere bildgebende Methode mit Radioisotopen ist die Positronen-Emissions-Tomographie, die positronenmittierende Isotope z.B. $^{43}$Sc, $^{44}$Sc, $^{52}$Fe, $^{55}$Co und $^{68}$Ga verwendet (Heis, W.D. Phelps, M.E. Position Emission Tomography of Brain, Springer Verlag Berlin, Heidelberg, New York, 1983).
Die erfindungsgemäßen Verbindungen können auch in der Radioimmunotherapie verwendet werden. Diese unterscheidet sich von der entsprechenden Diagnostik nur durch die Menge und Art des verwendeten radioaktiven Isotops. Ziel ist dabei die Zerstörung von Tumorzellen durch energiereiche kurzwellige Strahlung mit einer möglichst geringen Reichweite. Die Spezifität des erfindungsgemäßen Komplexes ist dabei von entscheidender Bedeutung, da unspezifisch lokaliserte Komplexe zur Zerstörung von gesundem Gewebe führen.
Am Zielort emittiert das wegen seiner zelltötenden Eigenschaften ausgewählte Metallion Strahlen, die die Zellen letal schädigen. Geeignete β-emittierende Ionen sind zum Beispiel $^{46}$Sc, $^{47}$Sc, $^{48}$Sc, $^{72}$Ga und $^{73}$Ga. Geeignete geringe Halbwertzeiten aufweisende α-emittierende Ionen sind zum Beispiel $^{211}$Bi, $^{212}$Bi. $^{213}$Bi und $^{214}$Bi , wobei $^{212}$Bi bevorzugt ist. Ein geeignetes Photonen- und Elektronen emittierendes Ion ist 158Gd, das aus 157Gd durch Neutroneneinfang erhalten werden kann.
Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung und dem benutzten Metallion.

Insgesamt ist es gelungen, neue Komplexverbindungen zu synthetisieren, die neue Möglichkeiten für die Diagnostik und Therapie erschließen. Die nachfolgenden Beispiele dienen der näheren Erläuterung des Erfindungsgegenstandes ohne ihn auf diese beschränken zu wollen.

**Beispiel 1**

a) 5,10,15,20-Tetrakis-{4-[(1-methylethyl)-oxycarbonylmethoxy]-phenyl}-porphyrin

3,21 g (4,73 mmol) 5,10,15,20-Tetrakis-(4-hydroxyphenyl)-porphyrin [CA RN 51094-17-8] werden in 250 ml wasserfreiem Dimethylformamid gelöst und mit 13,07 g (94,60 mmol) Kaliumcarbonat und 3,14 g (18,92 mmol) Kaliumiodid versetzt. Zu der unter Argon bei 21°C gerührten Suspension tropft man 17,13 g (94,60 mmol) Bromessigsäureisopropylester zu. Nach 24stündigem Rühren unter Lichtausschluß wird die Suspension filtriert, das Filtrat im Vakuum weitgehend eingedampft, mit 250 ml Dichlormethan versetzt und zweimal mit Wasser ausgeschüttelt. Die organische Phase wird über wasserfreiem Calciumchlorid getrocknet, filtriert und eingedampft. Der Rückstand wird an Kieselgel 60 (Merck) chromatographiert, das eingedampfte Eluat der Produktfraktionen aus Dichlormethan/Isopropanol umkristallisiert.
Ausbeute: 4,51 g (88 % d. Th.) violette Kristalle

| Analyse (bezogen auf wasserfreie Substanz): | | | | |
|---|---|---|---|---|
| C 71,23<br>C 71,11 | H 5,79<br>H 5,81 | N 5,19<br>N 5,08 | O 17,79 | ber. |

b) Mangan(III)-{5,10,15,20-Tetrakis-[4-[(1-methylethyl)-oxycarbonylmethoxy]-phenyl]-porphyrin}-acetat

3,04 g (2,82 mmol) der unter Beispiel la hergestellten Verbindung und 15,00 g Mangan(II)acetat-Tetrahydrat werden in 280 ml Essigsäure eine Stunde lang unter Rückfluß erhitzt. Man dampft im Vakuum bis zur Trockne ein, nimmt den Rückstand in 250 ml Dichlormethan auf und schüttelt einmal mit Wasser und zweimal mit gesättigter wässriger Natriumhydrogencarbonatlösung aus. Die organische Phase wird über wasserfreiem Magnesiumsulfat getrocknet, filtriert und eingedampft. Der Rückstand wird aus Isopropanol umkristallisiert.
Ausbeute: 3,06 g (91 % d. Th.) dunkelgrüne Kristalle

| Analyse (bezogen auf wasserfreie Substanz): | | | | | |
|---|---|---|---|---|---|
| C 66,55<br>C 66,38 | H 5,33<br>H 5,24 | Mn 4,61<br>Mn 4,59 | N 4,70<br>N 4,54 | O 18,80 | ber. |

c) Mangan(III)-{5,10,15,20-Tetrakis-[4-(carboxymethoxy)-phenyl]-porphyrin}-chlorid

2,52 g (2,12 mmol) der unter Beispiel 1b hergestellten Verbindung werden in 250 ml Tetrahydrofuran gelöst. Man gibt 250 ml zweinormale Natronlauge zu und kocht bis zur vollständigen Verseifung (ca. eine Stunde) unter Rückfluß. Dann dampft man das organische Lösungsmittel im Vakuum weitgehend ab und fällt durch Zugabe von halbkonzentrierter Salzsäure das Produkt durch Einstellen von pH 1-2 aus. Die Titelverbindung wird abgesaugt, mit Wasser gewaschen und im Vakuum getrocknet.
Ausbeute: 2,05 g (97 % d. Th.) dunkelgrüne Kristalle

| Analyse (bezogen auf wasserfreie Substanz): | | | | | | |
|---|---|---|---|---|---|---|
| C 62,50<br>C 62,41 | H 3,63<br>H 3,58 | Cl 3,55<br>Cl 3,71 | Mn 5,50<br>Mn 5,44 | N 5,61<br>N 5,42 | O 19,21 | ber. |

d) Mangan(III)-{5,10,15,20-Tetrakis-[4-(carboxylatomethoxy)-phenyl]-porphyrin}-chlorid, Tetranatriumsalz

1,52 g (1,52 mmol) der unter Beispiel 1c hergestellten Verbindung werden in 250 ml 2n Natronlauge 30 Minuten unter Rückfluß erhitzt. Nach dem Abkühlen wird mit halbkonzentrierter Salzsäure pH 9 eingestellt, das schwerlösliche Tetranatriumsalz abfiltriert, mit Wasser gewaschen und im Vakuum getrocknet.
Ausbeute: 1,47 g (89 % d. Th.) dunkelgrüne Kristalle

| Analyse (bezogen auf wasserfreie Substanz): | | | | | | | |
|---|---|---|---|---|---|---|---|
| C 57,45<br>C 57,38 | H 2,97<br>H 2,88 | Cl 3,26<br>Cl 3,47 | Mn 5,05<br>Mn 4,97 | N 5,15<br>N 5,03 | Na 8,46<br>Na 8,71 | O 17,66 | ber. |

**Beispiel 2**

a) 5,10,15,20-Tetrakis-{3-[1-methylethyl)-oxycarbonylmethoxy]-phenyl}-porphyrin

4,12 g (6,07 mmol) 5,10,15,20-Tetrakis-(3-hydroxyphenyl)-porphyrin [CA RN 22112-79-4] werden in 250 ml wasserfreiem N,N Dimethylformamid gelöst und mit 16,78 g (121,4 mmol) Kaliumcarbonat und 4,03 g (24,28 mmol) Kaliumiodid versetzt. Zu der unter Argon bei 21°C gerührten Suspension tropft man 21,98 g (121,4 mmol) Bromessigsäureisopropylester zu. Nach 24stündigem Rühren unter Lichtausschluß wird die Suspension filtriert, das Filtrat im Vakuum weitgehend eingedampft, mit 250 ml Dichlormethan versetzt und zweimal mit Wasser ausgeschüttelt. Die organische Phase wird über wasserfreiem Magnesiumsulfat getrocknet, filtriert und eingedampft. Der Rückstand wird an Kieselgel 60 chromatographiert, das eingedampfte Eluat aus Isopropanol umkristallisiert.
Ausbeute: 5,44 g (83 % d. Th.) violette Kristalle

| Analyse (bezogen auf wasserfreie Substanz): | | | | |
|---|---|---|---|---|
| C 71,23<br>C 71,08 | H 5,79<br>H 5,85 | N 5,19<br>N 5,12 | O 17,79 | ber. |

b) Mangan(III)-{5,10,15,20-Tetrakis-[3-[(1-methylethyl)-oxycarbonylmethoxy]-phenyl]-porphyrin}-acetat

5,09 g (4,72 mmol) der unter Beispiel 2a hergestellen Verbindung und 25,00 Mangan(II)acetat-Tetrahydrat werden in 200 ml Essigsäure eine Stunde lang unter Rückfluß erhitzt. Man dampft im Vakuum bis zur Trockne ein, nimmt den Rückstand in 250 ml Dichlormethan auf und schüttelt einmal mit Wasser und zweimal mit gesättigter wässriger Natriumhydrogencarbonatlösung aus. Die organische Phase wird über wasserfreiem Magnesiumsulfat getrocknet, filtriert und eingedampft. Der Rückstand wird aus Wasser/Isopropanol umkristallisiert.
Ausbeute: 5,00 g (89 % d. Th.) dunkelgrüne Kristalle

| Analyse (bezogen auf wasserfreie Substanz): | | | | | |
|---|---|---|---|---|---|
| C 66,55<br>C 66,35 | H 5,33<br>H 5,41 | $M_N$ 4,61<br>Mn 4,72 | N 4,70<br>N 4,61 | O 18,80 | ber. |

c) Mangan(III)-{5,10,15,20-Tetrakis-[3-(carboxymethoxy)-phenyl]-porphyrin}-chlorid

4,08 g (3,43 mmol) der unter Beispiel 2b hergestellten Verbindung werden in 200 ml Tetrahydrofuran gelöst. Man gibt 200 ml zweinormale wäßrige Natronlauge zu und kocht bis zur vollständigen Verseifung (ca. 1 Stunde) unter Rückfluß. Dann dampft man das organische Lösungsmittel im Vakuum weitgehend ab und fällt durch Zugabe von halbkonzentrierter Salzsäure das Produkt durch Einstellen von pH 1-2 aus. Die Titelverbindung wird abgesaugt, mit Wasser gewaschen und im Vakuum getrocknet.
Ausbeute: 3,18 g (93 % d. Th.) dunkelgrüne Kristalle

| Analyse (bezogen auf wasserfreie Substanz): | | | | | | |
|---|---|---|---|---|---|---|
| C 62,50<br>C 62,32 | H 3,63<br>H 3,75 | Cl 3,55<br>Cl 3,81 | Mn 5,50<br>Mn 5,41 | N 5,61<br>N 5,36 | O 19,21 | ber. |

d) Mangan(III)-{5,10,15,20-Tetrakis-[3-(carboxylatomethoxy)-phenyl]-porphyrin}-chlorid, Tetranatriumsalz

3,04 g (3,04 mmol) der unter 2c hergestellten Verbindung werden in 60,84 ml 0,20 normaler wässriger Natronlauge im Ultraschallbad gelöst. Die Lösung wird anschließend gefriergetrocknet.
Ausbeute: 3,31 g (100 % d. Th.) grünes Lyophilisat

| Analyse (bezogen auf wasserfreie Substanz): | | | | | | | |
|---|---|---|---|---|---|---|---|
| C 57,45<br>C 57,57 | H 2,97<br>H 3,14 | Cl 3,26<br>Cl 3,43 | Mn 5,05<br>Mn 4,92 | N 5,15<br>N 5,06 | Na 8,46<br>Na 8,55 | O 17,66 | ber. |

**Beispiel 3**

a) Zn(II)-[5,10,15,20-Tetrakis-{2-[(1-methylethyl)-oxycarbonylmethyl]-phenyl}-porphyrin], (4 Atropisomere)

33,34 g (0,150 mol) (2-Formylphenoxy)-essigsäureisopropylester (CA RN 51336-26-6) werden in 350 ml Propionsäure gelöst und mit 8,78 g (0,040 mol) Zink(II)acetat-Dihydrat versetzt. Man erwärmt die Lösung auf 60°C und läßt langsam 10,06 g (0,150 mol) Pyrrol zutropfen. Dann erhöht man die Temperatur auf 110°C und läßt eine Stunde weiterrühren. Nach dem Abkühlen wird im Vakuum vollständig eingedampft, der Rückstand in Dichlormethan aufgenommen und einmal mit Wasser und zweimal mit wässriger Natriumhydrogencarbonatlösung ausgeschüttelt. Die organische Phase wird über wasserfreiem Magnesiumsulfat getrocknet, filtriert, weitgehend eingedampft und an Kieselgel 60 (Merck) mit Dichlormethan/Methanol chromatographiert. Man erhält nach dem Eindampfen ein Gemisch von je vier Atropisomeren des Zinkkomplexes und des freien Liganden. Das Gemisch wird in 100 ml Dichlormethan gelöst, mit 10 ml konzentrierter methanolischer Zink(II)acetat-Dihydrat-Lösung versetzt und 30 Minuten unter Rückfluß gekocht. Nach dem Abkühlen gibt man weiteres Dichlormethan zu, schüttelt zweimal mit Wasser aus, trennt die organische Phase ab, trocknet diese über Magnesiumsulfat, filtriert und dampft ein. Der Rückstand wird aus Wasser/Isopropanol umkristallisiert.
Ausbeute: 10,41 g (24 % d. Th.) dunkelviolette Kristalle

| Analyse (bezogen auf wasserfreie Substanz): | | | | | |
|---|---|---|---|---|---|
| C 67,28<br>C 67,11 | H 5,29<br>H 5,48 | N 4,90<br>N 4,67 | O 16,80 | Zn 5,72<br>Zn 5,60 | ber. |

b) 5,10,15,20-Tetrakis-{2-[(1-methylethyl)-oxycarbonylmethoxy]-phenyl}-porphyrin (4 Atropisomere)

5,02 g (4,39 mmol) der unter Beispiel 2a) hergestellten Verbindung werden in einem Gemisch aus 60 ml Trifluoressigsäure, 10 ml Isopropanol und 10 ml Wasser ca. eine Stunde lang bei 20°C gerührt. Anschließend die Lösung bei 0°C mit gesättigter wässriger Natriumhydrogencarbonauflösung auf pH 7 gebracht und mit Dichlormethan ausgeschüttelt. Nach Trocknen der organischen Phase über wasserfreiem Magnesiumsulfat, Filtrieren und Eindampfen des Filtrats im Vakuum wird der resultierende Rückstand aus Isopropanol umkristallisiert.
Ausbeute: 4,22 g (89 % d. Th.) violette Kristalle

| Analyse (bezogen auf wasserfreie Substanz): | | | | |
|---|---|---|---|---|
| C 71,23<br>C 70,96 | H 5,79<br>H 5,88 | N 5,19<br>N 5,02 | O 17,79 | ber. |

c) Mangan(III)-[5,10,15,20-Tetrakis-{2-[(1-methylethyl)-oxycarbonylmethoxy]-phenyl)-porphyrin]-acetat (4 Atropisomere)

Die Darstellung der Titelverbindung kann analog der in Beispiel 1b oder 2b beschriebenen Methode ausgehend von 3b erfolgen, aber auch direkt aus dem unter Beispiel 3a beschreibenen Zinkkomplex:
4,27 g (3,74 mmol) der unter Beispiel 3a beschriebenen Verbindung werden 250 ml Essigsäure nach Zugabe von 20,00 g Mangan(II)acetat eine Stunde lang unter Rückfluß erhitzt. Man dampft im Vakuum bis zur Trockne ein, nimmt den Rückstand in 300 ml Dichlormethan auf und schüttelt einmal mit Wasser und zweimal mit gesättigter wässriger Natriumhydrogencarbonatlösung aus. Die organische Phase wird über wasserfreiem Magnesiumsulfat getrocknet, filtriert und eingedampft. Der Rückstand wird aus Isopropanol/Wasser umkristallisiert.
Ausbeute: 3,74 g (84 % d. Th.) dunkelgrüne Kristalle

| Analyse (bezogen auf wasserfreie Substanz): | | | | |
|---|---|---|---|---|
| C 66,55 | H 5,33 | Mn 4,61 | N 4,70 | O 18,80 |

(fortgesetzt)

| Analyse (bezogen auf wasserfreie Substanz): | | | | |
|---|---|---|---|---|
| C 66,42 | H 5,56 | Mn 4,73 | N 4,45 | |

d) Mangan(III)-{5,10,15,20-Tetrakis-(2-(carboxymethoxy)-phenyl]-porphyrin}-chlorid, (4 Atropisomere)

3,11 g (2,61 mmol) der unter Beispiel 3c hergestellten Verbindung werden in 250 ml Tetrahydrofuran gelöst. Man gibt 250 ml zweinormale wäßrige Natronlauge zu und kocht bis zur vollständigen Verseifung (ca. eine Stunde) unter Rückfluß. Dann dampft man das organische Lösemittel im Vakuum weitgehend ab und fällt durch Zugabe von halbkonzentrierter wässriger Salzsäure das Produkt durch Einstellen von pH 1-2 aus. Die Titelverbindung wird abgesaugt, mit Wasser gewaschen und im Vakuum getrocknet.
Ausbeute: 2,30 g (88 % d. Th.) dunkelgrüne Kristalle

| Analyse (bezogen auf wasserfreie Substanz): | | | | | |
|---|---|---|---|---|---|
| C 62,50<br>C 62,29 | H 3,63<br>H 3,75 | Cl 3,55<br>Cl 3,67 | Mn 5,50<br>Mn 5,53 | N 5,61<br>N 5,38 | O 19,21 |

**Beipiel 4**

a) Zink(II)-{5,10,15,20)-Tetrakis-[3,4-bis-(ethoxycarbonylmethoxy)-phenyl]-porphyrin}

10,11 g (32,58 mmol) 3,4-Bis-(ethoxycarbonylmethoxy)-benzaldehyd (CA RN 119309-58-9) und 1,79 g (8,15 mmol) Zinkacetat-Dihydrat werden in 100 ml Propionsäure gelöst. Zu der bei 80°C gerührten Lösung tropft man langsam 2,19 g (32,58 mmol) Pyrrol zu, das nunmehr dunkle Reaktionsgemenge läßt man eine Stunde bei 100°C weiterrühren. Nach dem Abkühlen wird im Vakuum vollständig eingedampft, der Rückstand in Dichlormethan aufgenommen und einmal mit Wasser und zweimal mit wässriger Natriumhydrogencarbonatlösung ausgeschüttelt. Die organische Phase wird über wasserfreiem Magnesiumsulfat getrocknet, filtriert, weitgehend eingedampft und an Kieselgel 60 (Merck) mit Dichlormethan/Methanol chromatographiert. Man erhält nach dem Eindampfen ein Gemisch aus dem Zinkkomplex und dem freien Liganden. Das Gemisch wird in 50 ml Dichlormethan gelöst, mit 5 ml konzentrierter methanolischer Zinkacetat-Dihydrat-Lösung versetzt und 30 Minuten unter Rückfluß gekocht. Nach dem Abkühlen gibt man weiteres Methylenchlorid zu, schüttelt zweimal mit Wasser aus, trennt die organische Phase ab, trocknet diese über Magnesiumsulfat, filtriert und dampft ein. Der Rückstand wird aus Wasser/Isopropanol umkristallisiert.
Ausbeute: 2,08 g (17 % d.Th.) dunkelviolette Kristalle

| Analyse (bezogen auf wasserfreie Substanz): | | | | | |
|---|---|---|---|---|---|
| C 61,07<br>C 60,84 | H 5,12<br>H 5,27 | N 3,75<br>N 3,68 | O 25,69 | Zn 4,37<br>Zn 4,09 | ber. |

b) 5,10,15-20-Tetrakis-[3,4-bis-(ethoxycarbonylmethoxy)-phenyl]-porphyrin

0,51 g (0,34 mmol) der unter Beispiel 4a) hergestellten Verbindung werden in einem Gemisch aus 10 ml Trifluoressigsäure, 1 ml Ethanol und 1 ml Wasser ca. eine Stunde bei 20°C gerührt. Anschließend wird die Lösung bei 0°C mit gesättigter wässriger Natriumhydrogencarbonatlösung auf pH 7 gebracht und mit Dichlormethan ausgeschüttelt. Nach Trocknen der organischen Phase über wasserfreiem Magnesiumsulfat, Filtrieren und Eindampfen des Filtrats im Vakuum wird der Rückstand aus Isopropanol umkristallisiert.
Ausbeute: 0,41 g (84 % d. Th.) violette Kristalle

| Analyse (bezogen auf wasserfreie Substanz): | | | | |
|---|---|---|---|---|
| C 63,77<br>C 63,62 | H 5,49<br>H 5,54 | N 3,91<br>N 3,84 | O 26,82 | ber. |

c) Mangan(III)-{5,10,15,20-Tetrakis-[3,4-bis-(ethoxycarbonylmethoxy)-phenyl]-porphyrin}-acetat

1,34 g (0,90 mmol) der unter Beispiel 4a) hergestellten Verbindung und 6,70 g Mangan(II) acetat-Tetrahydrat

werden in 125 ml Essigsäure eine Stunde lang unter Rückfluß erhitzt. Man dampft im Vakuum bis zur Trockne ein, nimmt den Rückstand in 125 ml Dichlormethan auf und schüttelt einmal mit Wasser zweimal mit gesättigter wässriger Natriumhydrogencarbonatlösung aus. Die organische Phase wird über wasserfreiem Magnesiumsulfat getrocknet, filtriert und eingedampft. Der Rückstand wird aus Isopropanol/Wasser umkristallisiert.
Ausbeute: 1,21 g (89 % d. Th.) dunkelgrüne Kristalle

| Analyse (bezogen auf wasserfreie Substanz): | | | | | | |
|---|---|---|---|---|---|---|
| C 60,06 | H 5,04 | Cl 2,33 | Mn 3,61 | N 3,69 | O 25,26 | ber. |
| C 59,82 | H 5,10 | Cl 2,14 | Mn 3,39 | N 3,48 | | |

d) Mangan(III)-{5,10,15,20-Tetrakis-[3,4-bis-(carboxymethoxy)-phenyl]-porphyrin}-chlorid

1,00 g (0,66 mmol) der unter Beispiel 4c) hergestellten Verbindung werden in 50 ml Tetrahydrofuran gelöst. Man gibt 50 ml zweinormale wäßrige Natronlauge zu und kocht bis zur vollständigen Verseifung (ca. eine Stunde) unter Rückfluß. Dann dampft man das organische Lösungsmittel im Vakuum weitgehend ab und fällt durch Zugabe halbkonzentrierter wässriger Salzsäure und Einstellen von pH 1-2 das Produkt. Die Titelverbindung wird abgesaugt mit Wasser gewaschen und im Vakuum getrocknet.
Ausbeute: 0,84 g (98 % d. Th.) dunkelgrüne Kristalle

| Analyse (bezogen auf wasserfreie Substanz): | | | | | | |
|---|---|---|---|---|---|---|
| C 55,63 | H 3,42 | Cl 2,74 | Mn 4,24 | N 4,33 | O 29,64 | ber. |
| C 55,74 | H 3,58 | Cl 2,92 | Mn 3,98 | N 4,12 | | |

e) Mangan(III)- {5,10,15,20-Tetrakis-[3,4-bis-(carboxylatomethoxy)-phenyl]-porphyrin}-chlorid, Octanatriumsalz

0,61 g (0,47 mmol) der unter Beispiel 4d) hergestellten Verbindung werden in 2,35 ml 0,20 normaler wässriger Natronlauge gelöst. Die Lösung wird anschließend gefriergetrocknet.
Ausbeute: 0,69 g (100 % d. Th.) grünes Lyophilisat

| Analyse (bezogen auf wasserfreie Substanz): | | | | | | | |
|---|---|---|---|---|---|---|---|
| C 48,98 | H 2,47 | Cl 2,41 | Mn 3,72 | N 3,81 | Na 12,50 | O 26,10 | ber. |
| C 48,87 | H 2,51 | Cl 2,56 | Mn 3,57 | N 3,72 | Na 12,72 | | |

**Beispiel 5**

a) 5,10,15,20-Tetrakis-(3-Acetylaminophenyl)-porphyrin

16,32 g (0,10 mol) 3-Acetylaminobenzaldehyd (EMS-Dottikon AG) werden in 300 ml Propionsäure gelöst und die Lösung auf 60°C erwärmt. Man läßt langsam 6,71 g (0,10 mol) Pyrrol zutropfen und rührt die dunkle Lösung eine Stunde bei 110°C. Nach dem Abkühlen wird im Vakuum vollständig eingedampft und der Rückstand aus Pyridin/Diethylether umkristallisiert.
Ausbeute: 8,43 g (40 % d. Th.) violette Kristalle

| Analyse (bezogen auf wasserfreie Substanz): | | | | |
|---|---|---|---|---|
| C 74,09 | H 5,02 | N 13,29 | O 7,59 | ber. |
| C 73,88 | H 5,11 | N 13,14 | | |

b) 5,10,15,20-Tetrakis-{{N-acetyl-N-[(1-methylethyl)-oxycarbonylmethyl]}-3-aminophenyl}-porphyrin

7,21 g (8,55 mmol) der unter Beispiel 5a) hergestellten Verbindung gelöst und mit 23,64 g (171,1 mmol) Kaliumcarbonat und 5,68 g (34,2 mmol) Kaliumiodid versetzt. Zu der unter Argon bei 21°C gerührten Lösung tropft man 30,97 g (171,1 mmol) Bromessigsäureisopropylester zu. Nach 36 h Rühren unter Lichtausschluß wird die Suspension filtriert, das Filtrat im Vakuum weitgehend eingedampft, mit 500 ml Dichlormethan versetzt und zweimal mit Wasser ausgeschüttelt. Die organische Phase wird über wasserfreiem Magnesiumsulfat getrocknet, filtriert und eingedampft. Der

Rückstand wird an Kieselgel 60 (Merck) chromatographiert, das eingedampfte Eluat aus Isopropanol umkristallisiert.
Ausbeute: 6,59 g (62 % d. Th.) violette Kristalle

| Analyse (bezogen auf wasserfreie Substanz): | | | | |
|---|---|---|---|---|
| C 69,55 | H 6,00 | N 9,01 | O 15,44 | ber. |
| C 69,71 | H 6,09 | N 8,86 | | |

c) Mangan-(III)-{5,10,15,20-Tetrakis-{{N-acetyl-N-[(1-methylethyl)-oxycarbonylmethyl]}-3-aminophenyl}-porphyrin}-acetat

6,07 g (4,88 mmol) der unter Beispiel 5b) hergestellten Verbindung und 30,0 g Mangan(II)-acetat-Tetrahydrat werden in 250 ml Essigsäure eine Stunde lang unter Rückfluß erhitzt. Man dampft im Vakuum bis zur Trockne ein, nimmt den Rückstand in 300 ml Dichlormethan auf und schüttelt einmal mit Wasser und zweimal mit gesättigter, wässriger Natriumhydrogencarbonatlösung aus. Die organische Phase wird über wasserfreiem Magnesiumsulfat getrocknet, filtriert und eingedampft. Der Rückstand wird aus wässrigem Isopropanol umkristallisiert.
Ausbeute: 5,98 g (90 % d. Th.) dunkelgrüne Kristalle

| Analyse (bezogen auf wasserfreie Substanz): | | | | | |
|---|---|---|---|---|---|
| C 65,58 | H 5,58 | Mn 4,05 | N 8,27 | O 16,53 | ber. |
| C 65,39 | H 5,67 | Mn 4,11 | N 8,18 | | |

d) Mangan(III)-{5,10,15,20-Tetrakis-[(N-acetyl-N-carboxymethyl)-aminophenyl]-3-porphyrin}-chlorid

4,67 g (3,45 mmol) der unter Beispiel 5c) hergestellten Verbindung werden in 200 ml Tetrahydrofuran gelöst. Man gibt 200 ml zwei normale, wäßrige Natronlauge zu und kocht bis zur vollständigen Verseifung (ca. eine Stunde) unter Rückfluß. Dann dampft man das organische Lösungsmittel im Vakuum weitgehend ab und fällt durch Zugabe von halbkonzentrierter wässriger Salzsäure das Produkt durch Einstellen von pH 1-2 aus. Die Titelverbindung wird abgesaugt mit Wasser gewaschen und im Vakuum getrocknet.
Ausbeute: 3,89 g (97 % d. Th.) dunkelgrüne Kristalle

| Analyse (bezogen auf wasserfreie Substanz): | | | | | | |
|---|---|---|---|---|---|---|
| C 61,94 | H 4,16 | Cl 3,05 | Mn 4,72 | N 9,63 | O 16,50 | ber. |
| C 61,82 | H 4,25 | Cl 3,31 | Mn 4,66 | N 9,53 | | |

e) Mangan(III)-{5,10,15,20-Tetrakis-[(N-acetyl-N-carboxylatomethyl)-3-aminophenyl]-porphyrin}-chlorid, Tetranatriumsalz

2,57 g (2,21 mmol) der unter Beispiel 2d) hergestellten Verbindung werden in 44,18 ml 0,20 normaler, wässriger Natronlauge im Ultraschallbad gelöst. Die Lösung wird anschließend gefriergetrocknet.
Ausbeute: 2,76 g (100 % d. Th.) dunkelgrünes Lyophilisat

| Analyse (bezogen auf wasserfreie Substanz): | | | | | | | |
|---|---|---|---|---|---|---|---|
| C 57,59 | H 3,54 | Cl 2,83 | Mn 4,39 | N 8,95 | Na 7,35 | O 15,34 | ber. |
| C 57,38 | H 3,77 | Cl 2,95 | Mn 4,27 | N 8,76 | Na 7,51 | | |

**Beispiel 6**

a) 5,10,15,20-Tetrakis-[3-(methoxycarbonylmethyl)-phenyl]-porphyrin

17,82 g (0,10 mol) (3-Formylphenyl)-essigsäuremethylester (B RN 3231441) werden in 300 ml Propionsäure gelöst und die Lösung auf 60°C erwärmt. Man läßt langsam 6,71 g (0,10 mol) Pyrrol zutropfen und rührt die dunkle Lösung 1 h lang bei 110°C. Nach dem Abkühlen wird im Vakuum vollständig eingedampft, der Rückstand im Dichlormethan aufgenommen und mit gesättigter wäßriger Natriumhydrogencarbonatlösung ausgeschüttelt. Nach dem Trocknen der organischen Phase über Natriumsulfat wird filtriert und eingedampft und der Rückstand an Kieselgel 60 (Merck) mit

Dichlormethan/Methanol chromatographiert. Der Rückstand der eingedampften Produktfraktionen wird aus Isopropanol umkristallisiert.
Ausbeute: 6,10 g (27 % d. Th.)

| Analyse (bezogen auf wasserfreie Substanz): | | | | |
|---|---|---|---|---|
| C 74,49 | H 5,13 | N 6,20 | O 14,17 | ber. |
| C 74,61 | H 5,27 | N 6,14 | | |

b) Mangan(III)-{5,10,15,20-Tetrakis-[3-(methoxycarbonylmethyl)-phenyl]-porphyrin}-acetat

5,78 g (6,40 mmol) der unter Beispiel 6a) hergestellten Verbindung und 25 g Mangan(II)-acetat-Tetrahydrat werden in 250 ml Essigsäure 3 h bei 80°C gerührt. Man dampft im Vakuum bis zur Trockne ein, nimmt den Rückstand in 300 ml Dichlormethan auf und schüttelt einmal mit Wasser und zweimal mit gesättigter wäßriger Natriumhydrogencarbonatlösung aus. Die organische Phase wird über wasserfreiem Magnesiumsulfat getrocknet, filtriert und eingedampft. Der Rückstand wird aus wäßrigem Methanol umkristallisiert.
Ausbeute: 5,65 g (87 % d.Th.) dunkelgrüne Kristalle

| Analyse (bezogen auf wasserfreie Substanz): | | | | | |
|---|---|---|---|---|---|
| C 68,64 | H 4,67 | Mn 5,41 | N 5,52 | O 15,76 | ber. |
| C 68,53 | H 5,79 | Mn 5,58 | N 5,42 | | |

c) Mangan(III)-{5,10,15,20-Tetrakis-[3-(carboxymethyl)-phenyl]-porphyrin}-chlorid

4,28 g (4,22 mmol) der unter Beispiel 6b) hergestellten Verbindung werden in 200 ml Tetrahydrofuran gelöst. Man gibt 200 ml zweinormale, wäßrige Natronlauge zu und kocht bis zur vollständigen Verseifung (ca. eine Stunde) unter Rückfluß. Dann dampft man das organische Lösungsmittel im Vakuum weitgehend ab und fällt durch Zugabe von halbkonzentrierter wässriger Salzsäure bei pH 1-2 das Produkt aus. Die Titelverbindung wird abgesaugt mit Wasser gewaschen und im Vakuum getrocknet.
Ausbeute: 3,75 g (95 % d. Th.) dunkelgrüne Kristalle

| Analyse (bezogen auf wasserfreie Substanz): | | | | | |
|---|---|---|---|---|---|
| C 66,78 | H 3,88 | Cl 3,79 | Mn 5,87 | N 5,99 | O 13,69 |
| C 66,77 | H 4,00 | Cl 3,91 | Mn 5,73 | N 5,92 | |

**Beispiel 7**

a) 5,10,15,20-Tetrakis-{3-[2-(ethoxycarbonyl)-ethyl]-phenyl}-porphyrin

20,62 g (0,10 mol) 3-{3-Formylphenyl)-propionsäureethylester (CARN 110114-05-1) werden in 300 ml Propionsäure gelöst und die Lösung auf 60°C erwärmt. Man läßt langsam 6,71 (0,10 mol) Pyrrol zutropfen und rührt die dunkle Lösung 1 h lang bei 110°C. Nach dem Abkühlen wird im Vakuum vollständig eingedampft, der Rückstand in Dichlormethan aufgenommen und mit gesättigter wäßriger Natriumhydrogencarbonatlösung ausgeschüttelt. Nach dem Trocknen der organischen Phase über Natriumsulfat wird filtriert und eingedampft und der Rückstand an Kieselgel 60 (Merck) mit Dichlormethan/Ethanol chromatographiert. Der Rückstand der eingedampften Produktfraktionen wird aus Isopropanol umkristallisiert.
Ausbeute: 8,12 g (32 % d. Th.) violette Kristalle

| Analyse (bezogen auf wasserfreie Substanz): | | | | |
|---|---|---|---|---|
| C 75,72 | H 6,16 | N 5,52 | O 12,61 | ber. |
| C 75,81 | H 6,09 | N 5,57 | | |

b) Mangan(III)-{5,10,15,20-Tetrakis-{3-[2-(ethoxycarbonyl)-ethyl]-phenyl}-porphyrin}-acetat

7,32 g (7,21 mmol) der unter Beispiel 7a) hergestellten Verbindung und 28 g Mangan(II)-acetat-Tetrahydrat werden

in 250 ml Essigsäure 3 h bei 80°C gerührt. Man dampft im Vakuum ein, nimmt den Rückstand in 300 ml Dichlormethan auf und schüttelt einmal mit Wasser und zweimal mit gesättigter, wäßriger Natriumhydrogencarbonatlösung aus. Die organische Phase wird über wasserfreiem Natriumsulfat getrocknet, filtriert und eingedampft. Der Rückstand wird aus wässrigem Ethanol umkristallisiert.
Ausbeute: 7,64 g (94 % d. Th.) dunkelgrüne Kristalle

| Analyse (bezogen auf eine wasserfreie Substanz): | | | | | |
|---|---|---|---|---|---|
| C 70,33<br>C 70,18 | H 5,63<br>H 5,71 | Mn 4,87<br>Mn 4,80 | N 4,97<br>N 4,82 | O 14,19 | ber. |

c) Mangan(III)-{5,10,15,20-Tetrakis-[3-(2-carboxyethyl)-phenyl]-porphyrin}-chlorid

6,81 g (6,04 mmol) der unter Beispiel 7b hergestellten Verbindung werden in 200 ml Tetrahydrofuran gelöst. Man gibt 200 ml zweinormale, wäßrige Natronlauge zu und kocht bis zur vollständigen Verseifung (ca. eine Stunde) unter Rückfluß. Dann dampft man das organische Lösungsmittel im Vakuum weitgehend ab und fällt durch Zugabe von halbkonzentrierter Salzsäure bei pH 1-2 das Produkt aus. Die Titelverbindung wird abgesaugt, mit Wasser gewaschen und im Vakuum getrocknet.
Ausbeute: 5,81 g (97 % d. Th.) dunkelgrüne Kristalle

| Analyse (bezogen auf eine wasserfreie Substanz): | | | | | | |
|---|---|---|---|---|---|---|
| C 67,85<br>C 67,69 | H 4,47<br>H 4,53 | Cl 3,58<br>Cl 3,72 | Mn 5,54<br>Mn 5,38 | N 5,65<br>N 5,52 | O 12,91 | ber. |

**Beispiel 8**

a) 2-{N,N-Bis-[(1-methylethyl)-oxycarbonylmethyl]-3-aminophenyl}-1,3-dioxolan

165,19 g (1,00 mol) 2-(3-Aminophenyl)-1,3-dioxolan (CA 108 (15) : 131815 r), 156,35 g (3,00 mol) gepulvertes Kaliumhydroxid und 11,39 g (0,05 mol) Benzyltriethylammoniumchlorid werden unter Argon kräftig mechanisch gerührt und auf 80°C erwärmt. Zu der Suspension läßt man 543,09 g (3,00 mol) Bromessigsäureisopropylester zutropfen und rührt das Reaktionsgemisch ca. 8 h bei 80°C. Dann wird filtriert, das Filtrat mehrmals mit konz. wäßriger Natriumhydrogencarbonatlösung ausgeschüttelt, die organische Phase über wasserfreiem Magnesiumsulfat getrocknet, filtriert und im Vakuum eingedampft. Der Rückstand wird an Kieselgel 60 (Merck) mit Methylenchlorid/Methanol chromatographiert. Die Produktfraktionen ergeben nach Eindampfen ein farbloses Öl.
Ausbeute: 288,69 g (79 % d. Th.)

| Analyse (bezogen auf wasserfreie Substanz): | | | | |
|---|---|---|---|---|
| C 62,45<br>C 62,39 | H 7,45<br>H 7,53 | N 3,83<br>N 3,84 | O 26,27 | ber. |

b) N,N-Bis-[(1-methylethyl)-oxycarbonylmethyl]-3-aminobenzaldehyd

200,00 g (0,55 mol) der unter Beispiel 8a) hergestellten Verbindung werden in 1 l eines 1 : 1-Gemisches aus Aceton und zweinormaler wäßriger Salzsäure bis zur vollständigen Umsetzung bei 50°C unter Argon gerührt. Dann wird im Vakuum eingedampft, der Rückstand in Dichlormethan aufgenommen und zweimal mit gesättigter wäßriger Natriumhydrogencarbonatlösung ausgeschüttelt. Die organische Phase wird über wasserfreiem Magnesiumsulfat getrocknet, filtriert und im Vakuum eingedampft. Man erhält das Produkt als öligen Rückstand.
Ausbeute: 160,06 g (91 % d. Th.)

| Analyse (bezogen auf wasserfreie Substanz): | | | | |
|---|---|---|---|---|
| C 63,54<br>C 63,49 | H 7,21<br>H 7,47 | N 4,36<br>N 4,22 | O 24,89 | ber. |

c) 5,10,15,20-Tetrakis-{N,N-bis-[(1-methylethyl)-oxycarbonylmethyl]-3-aminophenyl}-porphyrin

32,14 g (0,10 mol) der unter Beispiel 8b hergestellten Verbindung werden in 400 ml Propionsäure gelöst. Man erwärmt die Lösung auf 60°C, läßt langsam 6,71 g (0,10 mol) Pyrrol zutropfen und rührt die dunkle Lösung 1 Stunde lang bei 110°C. Nach dem Abkühlen wird im Vakuum vollständig eingedampft, der Rückstand mit Dichlormethan aufgenommen und mit gesättigter wäßriger Natriumhydrogencarbonatlösung ausgeschüttelt. Nach dem Trocknen der organischen Phase über Natriumsulfat wird filtriert, eingedampft und der Rückstand an Kieselgel 60 (Merck) mit Dichlormethan/Methanol chromatographiert. Der Rückstand der eingedampften Produktfraktionen wird aus wäßrigem Isopropanol umkristallisiert.
Ausbeute: 9,22 g (25 % d. Th.) violette Kristalle

| Analyse (bezogen auf wasserfreie Substanz): | | | | |
|---|---|---|---|---|
| C 68,37 | H 6,69 | N 7,59 | O 17,35 | ber. |
| C 68,44 | H 6,52 | N 7,63 | | |

d) Mangan(III)-{5,10,15,20-Tetrakis-{N,N-bis-[(1-methylethyl)-oxycarbonylmethyl]-3-aminophenyl}-porphyrin}-acetat

8,71 g (5,90 mmol) der unter Beispiel 8c) hergestellten Verbindung und 30 g Mangan(II)-acetat-Tetrahydrat werden in 250 ml Essigsäure 2 Stunden bei 100°C gerührt. Man dampft im Vakuum bis zur Trockne ein, nimmt den Rückstand in Methylenchlorid auf und schüttelt einmal mit Wasser und zweimal mit gesättigter wäßriger Natriumhydrogencarbonatlösung aus. Die organische Phase wird über wasserfreiem Magnesiumsulfat getrocknet, filtriert und eingedampft. Der Rückstand wird aus wäßrigem Isopropanol umkristallisiert.
Ausbeute: 8,81 g (94 % d. Th.) dunkelgrüne Kristalle

| Analyse (bezogen auf wasserfreie Substanz) | | | | | |
|---|---|---|---|---|---|
| C 65,06 | H 6,28 | Mn 3,46 | N 7,06 | O 18,14 | ber. |
| C 64,87 | H 6,42 | Mn 3,37 | N 6,91 | | |

e) Mangan(III)-{5,10,15,20-Tetrakis-{N,N-bis-(carboxymethyl)-3-aminophenyl-porphyrin}-chlorid

7,24 g (4,56 mmol) der unter Beispiel 8d hergestellten Verbindung werden in 200 ml Tetrahydrofuran gelöst. Man gibt 200 ml zweinormale wäßrige Natronlauge zu und kocht bis zur vollständigen Verseifung (ca. 1,5 Stunden) unter Rückfluß. Dann dampft man das organische Lösemittel im Vakuum weitgehend ab und fällt durch Zugabe von halbkonzentrierter wäßriger Salzsäure bei pH 1-2 das Produkt aus. Die Titelverbindung wird abgesaugt, mit Wasser gewaschen und im Vakuum getrocknet.
Ausbeute: 5,49 g (98 % d. Th.) dunkelgrüne Kristalle

| Analyse (bezogen auf wasserfreie Substanz): | | | | | | |
|---|---|---|---|---|---|---|
| C 58,71 | H 3,94 | Cl 2,89 | Mn 4,48 | N 9,13 | O 20,85 | ber. |
| C 58,55 | H 3,99 | Cl 2,96 | Mn 4,31 | N 9,06 | | |

**Beispiel 9**

a) 5,10,15,20-Tetrakis-[3-(2-methoxycarbonyl-2-hydroxyethoxy)-phenyl]-porphyrin

7,64 g (11,26 mmol) 5,10,15,20-Tetrakis-(3-hydroxyphenyl)-porphyrin [CA RN 22112-79-4] werden in 300 ml Wasser suspendiert. Man gibt 14,02 g (112,56 mmol) β-Chlormilchsäure (Sigma) zu und stellt durch Zutropfen von zweinormaler Natronlauge pH 9-10 ein. Nach 12 Stunden Rühren und Argon bei 40°C wird das Reaktionsprodukt durch Einstellen von pH 1-2 mit wässriger Salzsäure gefällt. Der Niederschlag wird abgesaugt im Vakuum getrocknet und in einem Gemisch aus Dichlormethan/Methanol gelöst. Dann tropft man unter Rühren bei 20°C bis zur dünnschichtchromatographisch festgestellten vollständigen Umsetzung etherische Diazomethanlösung zu. Das Reaktionsgemisch wird durch Zugabe von Essigsäure von überschüssigem Diazomethan befreit und im Vakuum eingedampft, der Rückstand an Kieselgel 60 (Merck) mit Dichlormethan/Methanol chromatographiert. Die Produktfraktionen werden eingedampft, der Rückstand aus wäßrigem Methanol umkristallisiert.
Ausbeute: 7,22 g (59 % d. Th.) violette Kristalle

| Analyse (bezogen auf wasserfreie Substanz): | | | | |
|---|---|---|---|---|
| C 66,29 | H 5,01 | N 5,15 | O 23,55 | ber. |
| C 66,14 | H 5,24 | N 5,03 | | |

b) Mangan(III)-{5,10,15,20-Tetrakis-[3-(2-methoxycarbonyl-2-hydroxyethoxy)-phenyl]-porphyrin}-acetat

5,89 g (5,42 mmol) der unter Beispiel 9a hergestellten Verbindung und 23,00 g Mangan(II)-acetat-Tetrahydrat werden in 200 ml Essigsäure 3 Stunden lang bei 80°C gerührt. Man dampft im Vakuum bis zur Trockne ein, nimmt den Rückstand in Dichlormethan auf und schüttelt einmal mit Wasser und zweimal mit gesättigter wäßriger Natriumhydrogencarbonatlösung aus. Die organische Phase wird über wasserfreiem Magnesiumsulfat getrocknet, filtriert und eingedampft. Der Rückstand wird aus wäßrigem Methanol umkristallisiert.
Ausbeute: 5,39 g (83 % d. Th.) dunkelgrüne Kristalle

| Analyse (bezogen auf wasserfreie Substanz): | | | | | |
|---|---|---|---|---|---|
| C 62,10 | H 4,62 | Mn 4,58 | N 4,67 | O 24,02 | ber. |
| C 62,23 | H 4,57 | Mn 4,44 | N 4,52 | | |

c) Mangan(III)-{5,10,15,20-Tetrakis-[3-(2-carboxy-2-hydroxyethoxy)-phenyl]-porphyrin}-chlorid

4,74 g (3,95 mmol) der unter Beispiel 9b hergestellten Verbindung werden in 200 ml Tetrahydrofuran gelöst. Man gibt 200 ml zweinormale wäßrige Natronlauge zu und rührt bis zur vollständigen Verseifung bei 60°C (ca. 3 Stunden). Dann dampft man das organische Lösemittel im Vakuum weitgehend ab und fällt das Produkt durch Zugabe von halbkonzentrierter Salzsäure bei pH 1-2 aus. die Titelverbindung wird abgesaugt, mit Wasser gewaschen und im Vakuum getrocknet.
Ausbeute: 3,89 g (88 % d. Th.) dunkelgrüne Kristalle

| Analyse (bezogen auf wasserfreie Substanz): | | | | | | |
|---|---|---|---|---|---|---|
| C 60,09 | H 3,96 | Cl 3,17 | Mn 4,91 | N 5,01 | O 22,87 | ber. |
| C 59,87 | H 4,08 | Cl 3,24 | Mn 4,81 | N 4,83 | | |

**Beispiel 10**

a) 5,10,15,20-Tetrakis-{N-[(1-methylethyl)-oxycarbonylmethyl]-N-(p-toluolsulfonyl)-3-aminomethylphenyl}-porphyrin

1.98 g (1,13 mmol) 5,10,15,20-Tetrakis-[3-(p-toluolsulfonamidomethyl)-phenyl]-porphyrin [T.C. Bruice et al., J. Am. Chem. Soc. 1991, 113, 4208-4218] werden in 20 ml wasserfreiem N,N-Dimethylformamid gelöst und mit 1,25 g (9,06 mmol) Kaliumcarbonat versetzt. Zu der unter Argon bei 21°C gerührten Suspension tropft man 1,64 g (9,06 mmol) Bromessigsäureisopropylester. Nach 24 h Rühren unter Lichtausschluß wird die Suspension filtriert, das Filtrat im Vakuum weitgehend eingedampft, mit 50 ml Dichlormethan versetzt und zweimals mit Wasser ausgeschüttelt. Die organische Phase wird über wasserfreiem Natriumsulfat getrocknet, filtriert und eingedampft. Der Rückstand wird an Kieselgel 60 (Merck) chromatographiert, das eingedampfte Eluat aus Isopropanol umkristallisiert.
Ausbeute: 1,64 g (82 % d. Th.) violette Kristalle

| Analyse (bezogen auf wasserfreie Substanz): | | | | | |
|---|---|---|---|---|---|
| C 65,96 | H 5,65 | N 6,41 | O 14,64 | S 7,34 | ber. |
| C 65,84 | H 5,79 | N 6,40 | | S 7,16 | |

b) Mangan(III)-{5,10,15,20-Tetrakis-{N-[(1-methylethyl)-oxycarbonylmethyl]-N-(p-toluolsulfonyl)-3-aminomethylphenyl}-porphyrin}-acetat

1,47 (0,84 mmol) der unter Beispiel 10a hergestellten Verbindung und 8,00 g Mangan(II)-acetat-Tetrahydrat werden in 200 ml Essigsäure eine Stunde lang unter Rückfluß gekocht. Man dampft im Vakuum bis zur Trockne ein, nimmt den Rückstand in 50 ml Dichlormethan auf und schüttelt einmal mit Wasser und zweimal mit gesättigter wässriger

Natriumhydrogencarbonatlösung aus. Die organische Phase wird über wasserfreiem Magnesiumsulfat getrocknet, filtriert und eingedampft. Der Rückstand wird aus Wasser/Isopropanol umkristallisiert.
Ausbeute: 1,19 g (75 % d. Th.) dunkelgrüne Kristalle

| Analyse (bezogen auf wasserfreie Substanz): | | | | | | |
|---|---|---|---|---|---|---|
| C 63,28<br>C 63,17 | H 5,36<br>H 5,37 | Mn 2,95<br>Mn 2,88 | N 6,02<br>M 5,86 | O 15,48 | S 6,90<br>S 6,79 | ber. |

c) Mangan(III)-{5,10,15,20-Tetrakis-[N-carboxymethyl)-N-(p-toluolsulfonyl)-3-aminomethylphenyl]-porphyrin}-chlorid

0,97 g (0,52 mmol) der unter Beispiel 10b hergestellten Verbindung werden in 50 ml Tetrahydrofuran gelöst. Man gibt 100 ml zweinormale wässrige Natronlauge zu und kocht bis zur vollständigen Verseifung (ca. eine Stunde) unter Rückfluß. Dann dampft man das organische Lösungsmittel im Vakuum weitgehend ab und fällt durch Zugabe von halbkonzentrierter wässriger Salzsäure das Produkt bei pH 1 - 2 aus. Die Titelverbindung wird abgesaugt, mit Wasser gewaschen und im Vakuum getrocknet.
Ausbeute: 0,86 g (99 % d. Th.) dunkelgrüne Kristalle

| Analyse (bezogen auf wasserfreie Substanz): | | | | | | |
|---|---|---|---|---|---|---|
| C 60,48<br>C 60,51 | H 4,35<br>H 4,47 | Cl 2,13<br>Cl 2,43 | Mn 3,29<br>Mn 3,08 | N 6,72<br>N 6,49 | O 15,35 | S 7,69<br>S 7,51 |

**Beispiel 11**

a) 5,10,15,20-Tetrakis-[3-(3-phtalimidopropyloxy)-phenyl]-porphyrin

7,56 g (11,14 mmol) 5, 10,15,20-Tetrakis-(3-hydroxyphenyl)-porphyrin [CARN 22112-79-4] werden in 300 ml wasserfreiem N,N-Dimethylformamid gelöst und mit 30,79 g (222,8 mmol) Kaliumcarbonat und 7,40 g (44,55 mmol) Kaliumiodid versetzt. Zu der unter Argon bei 21°C gerührten Suspension gibt man 59,72 g (222, 8 mmol) N-(3-Brompropyl)-phtalimid zu. Nach 24 h Rühren unter Lichtausschluß wird die Suspension filtriert, das Filtrat im Vakuum weitgehend eingedampft, mit Dichlormethan versetzt und zweimal mit Wasser ausgeschüttelt. Die organische Phase wird über wasserfreiem Magnesiumsulfat getrocknet, filtriert und eingedampft, der Rückstand an Kieselgel 60 (Merck) mit Dichlormethan/Methanol chromatographiert. Die Produktfraktionen werden eingedampft, in wenig Dichlormethan aufgenommen und die Titelverbindung durch Zugabe von Diethylether ausgefällt.
Ausbeute: 11,77 g (74 % d. Th) violette Kristalle

| Analyse (bezogen auf wasserfreie Substanz) | | | | |
|---|---|---|---|---|
| C 74,04<br>C 74,21 | H 4,66<br>H 4,49 | N 7,85<br>N 7,84 | O 13,45 | ber. |

b) 5,10,15,20-Tetrakis-[3-(-3-aminopropyloxy)-phenyl]-porphyrin

10,09 g (7,07 mmol) der unter Beispiel 11a hergestellten Verbindung werden in 300 ml Pyridin nach Zugabe von 3,54 g (70,68 mmol) Hydrazin-Hydrat bis zu vollständigen Deblockierung der Aminoreste (ca 1 h) unter Rückfluß gekocht. Dann wird im Vakuum eingedampft, der Rückstand in Dichlormethan aufgenommen und mehrmals mit gesättigter wässriger Natriumhydrogencarbonatlösung ausgeschüttelt. Die organische Phase wird wasserfreiem Magnesiumsulfat getrocknet, filtriert und eingedampft. Der Rückstand wird an Aluminiumoxid 90 (Merck) mit Dichlormethan/Methanol chromatographiert, die Produktfraktionen werden eingedampft, der Rückstand wird in wenig Dichlormethan aufgenommen, die Titelverbindung durch Zutropfen von tert. Butylmethylether ausgefällt und abgesaugt.
Ausbeute: 4,87 g (76 % d. Th.)

| Analyse (bezogen auf wasserfreie Substanz) | | | | |
|---|---|---|---|---|
| C 74,15<br>C 74,94 | H 6,44<br>H 6,41 | N 12,35<br>N 12,28 | O 7,05 | ber. |

c) 5,10,15,20-Tetrakis-[3-(N-acetyl-3-aminopropyloxy)-phenyl]-porphyrin

4,11 g (4,53 mmol) der unter Beispiel 11b hergestellten Verbindung werden in 150 ml eines 1 : 1-Gemisches aus Pyridin und Essigsäureanhydrid 8 h lang unter Argon bei 21°C gerührt. Dann wird im Vakuum eingedampft, der Rückstand in Dichlormethan aufgenommen und einmal mit gesättigter wässriger Natriumhydrogensulfatlösung und zweimal mit gesättigter wässriger Natriumhydrogencarbonatlösung ausgeschüttelt. Die organische Phase wird über Magnesiumsulfat getrocknet, filtriert, eingedampft und der Rückstand an Kieselgel 60 (Merck) mit Dichlormethan/Methanol chromatographiert. Die eingedampften Produktfraktionen werden aus Isopropanol umkristallisiert.
Ausbeute: 3,95 g (81 % d. Th.) violette Kristalle

| Analyse (bezogen auf wasserfreie Substanz) | | | | |
|---|---|---|---|---|
| C 71,49<br>C 71,31 | H 6,19<br>H 6,28 | N 10,42<br>N 10,24 | O 11,90 | ber. |

d) 5,10,15,20-Tetrakis-{3-{N-acetyl-N-[(1-methylethyl)-oxycarbonylmethyl]-3-aminopropyloxy}-phenyl}-porphyrin

3,57 g (3,32 mmol) der unter Beispiel 11c hergestellten Verbindung werden in 250 ml wasserfreiem N,N-Dimethylformamid gelöst und mit 9,18 g (66,4 mmol) Kaliumcarbonat und 2,20 g (13,3 mmol) Kaliumiodid versetzt. Zu der unter Argon bei 21°C gerührten Suspension tropft man 12,02 g (66,4 mmol) Bromessigsäureisopropylester zu. Nach 24 h Rühren unter Lichtausschluß wird die Suspension filtriert, das Filtrat im Vakuum weitgehend eingedampft, mit Dichlormethan versetzt und zweimal mit Wasser ausgeschüttet. Die organische Phase wird über wasserfreiem Magnesiumsulfat getrocknet, filtriert und eingedampft. Der Rückstand wird an Kieselgel 60 (Merck) mit Dichlormethan/Methanol chromatographiert und das eingedampfte Eluat aus Isopropanol umkristallisiert.
Ausbeute: 3,43 g (70 % d. Th.) violette Kristalle

| Analyse (bezogen auf wasserfreie Substanz) | | | | |
|---|---|---|---|---|
| C 68,37<br>C 68,30 | H 6,69<br>H 6,79 | N 7,59<br>N 7,33 | O 17,35 | ber. |

e) Mangan(III)-{5,10,15,20-Tetrakis-{3-{N-acetyl-N-[(1-methylethyl)-oxycarbonyl methyl]-3-aminopropyloxy}-phenyl}-porphyrin}-acetat

3,01 g (2,04 mmol) der unter Beispiel 11d hergestellten Verbindung und 15,00 g Mangan(II)-acetat-Tetrahydrat werden in 150 ml Essigsäure eine Stunde lang unter Rückfluß erhitzt. Man dampft im Vakuum bis zu Trockne ein, nimmt den Rückstand in 200 ml Dichlormethan auf und schüttelt einmal mit Wasser und zweimal mit gesättigter wässriger Natriumhydrogencarbonatlösung aus. Die organische Phase wird über wasserfreiem Magnesiumsulfat getrocknet, filtriert und eingedampft. Der Rückstand wird aus Wasser/Isopropanol umkristallisiert.
Ausbeute: 2,75 g (85 % d. Th.) dunkel grüne Kristalle

| Analyse (bezogen auf wasserfreie Substanz) | | | | | |
|---|---|---|---|---|---|
| C 65,06<br>C 65,12 | H 6,28<br>H 6,21 | Mn 3,46<br>Mn 3,39 | N 7,06<br>N 7,14 | O 18,14 | ber |

f) Mangan(III)- {5,10,15,20-Tetrakis-[{3-(N-acetyl-N-carboxylmethyl-3-aminopropyloxy)-phenyl]-porphyrin}-chlorid

2,68 g (1,69 mol) der unter Beispiel 11e hergestellten Verbindung werden in 150 ml Tetrahydrofuran gelöst. Man gibt 150 ml zweinormale wässrige Natronlauge zu und kocht bis zu vollständigen Verseifung (ca 1 h) unter Rückfluß. Dann dampft man das organische Lösungsmittel im Vakuum weitgehend ab und fällt durch Zugabe von halbkonzentrierter wässriger Salzsäure das Produkt bei pH 1 - 2 aus. Die Titelverbindung wird abgesaugt, mit Wasser gewaschen und im Vakuum getrocknet.
Ausbeute: 2,33 g (99 % d. Th.) dunkelgrüne Kristalle

| Analyse (bezogen auf wasserfreie Substanz) | | | | | | |
|---|---|---|---|---|---|---|
| C 61,96 | H 5,20 | Cl 2,54 | Mn 3,94 | N 8,03 | O 18,34 | ber. |

(fortgesetzt)

| Analyse (bezogen auf wasserfreie Substanz) | | | | | | |
|---|---|---|---|---|---|---|
| C 61,88 | H 5,45 | Cl 2,76 | Mn 3,79 | N 7,82 | | |

**Beispiel 12**

a) Eisen (III)-{5,10,15,20-Tetrakis-{3-[(1-methylethyl)-oxycarbonyl-methoxy]-phenyl}-porphyrin}-acetat

3,97 g (3,68 mmol) der unter Beispiel 2a hergestellten Verbindung und 8,0 g Eisen (II)-Sulfat-Heptahydrat werden in einem Gemisch aus 200 ml Essigsäure, 50 ml Pyridin und 10 ml Wasser 2 h lang unter Rückfluß erhitzt. Man dampft im Vakuum bis zur Trockne ein, nimmt den Rückstand in 200 ml Dichlormethan auf und schüttelt einmal mit Wasser und zweimal mit gesättigter wässriger Natriumhydrogencarbonatlösung aus. Die organische Phase wird über wasserfreiem Magnesiumsulfat getrocknet, filtriert und eingedampft. Der Rückstand wird aus Wasser/Isopropanol umkristallisiert.
Ausbeute: 3,95 g (90 % d. Th.) dunkelgrüne Kristalle

| Analyse (bezogen auf wasserfreie Substanz) | | | | | | |
|---|---|---|---|---|---|---|
| C 66,50<br>C 66,44 | H 5,33<br>H 5,51 | Fe 4,68<br>Fe 4,61 | Mn 3,94<br>Mn 3,79 | N 4,70<br>N 4,53 | O 18,79 | ber. |

b) Eisen (III)-{5,10,15,20-Tetrakis-[3-(carboxymethoxy)-phenyl]-porphyrin}-chlorid

3,33 g (2,79 mmol) der unter Beispiel 12a hergestellten Verbindung werden in 150 ml Tetrahydrofuran gelöst. Man gibt 150 ml zweinormale wässrige Natronlauge zu und kocht bis zur vollständigen Verseifung (ca. 1 h) unter Rückfluß. Dann dampft man das organische Lösungsmittel zum Vakuum weitgehend ab und fällt durch Zugabe von halbkonzentrierter Salzsäure das Produkt bei pH 1 - 2 aus. Die Titelverbindung wird abgesaugt mit Wasser gewaschen und im Vakuum getrocknet.
Ausbeute: 2,76 (99 % d. Th.) dunkelgrüne Kristalle

| Analyse (bezogen auf wasserfreie Substanz) | | | | | | |
|---|---|---|---|---|---|---|
| C 62,45<br>C 62,41 | H 3,61<br>H 3,74 | Cl 3,54<br>Cl 3,68 | Fe 4,68<br>Fe 4,61 | N 5,60<br>N 5,52 | O 19,20 | ber. |

**Beispiel 13**

a) 5,10,15,20-Tetrakis-[3-(carboxymethoxy)-phenyl]-porphyrin, Dihydrochlorid

4,68 g (4,34 mmol) der unter Beispiel 2a herstellten Verbindung werden in 100 ml Tetrahydrofuran gelöst. Man gibt 100 ml zweinormale wäßrige Natronlauge zu und kocht bis zur vollständigen Verseifung unter Rückfluß. Dann dampft man das organische Lösungsmittel im Vakuum weitgehend ab und fällt das Produkt durch Zugabe von halbkonzentrierter Salzsäure bei pH 1-2 aus. Die Titelverbindung wird abgesaugt, mit Wasser gewaschen und im Vakuum getrocknet.
Ausbeute: 4,22 g (99 % d. Th.) dunkelgrüne Kristalle

| Analyse (bezogen auf wasserfreie Substanz) | | | | | |
|---|---|---|---|---|---|
| C 63,48<br>63,37 | H 4,10<br>H 4,19 | Cl 7,21<br>Cl 7,04 | N 5,69<br>N 5,56 | O 19,52 | ber. |

b) 5,10,15,20-Tetrakis-[3-(chlorcarbonylmethoxy)-phenyl]-porphyrin, Dihydrochlorid

3,07 g (3,12 mmol) der nach Beispiel 13a hergestellten Verbindung werden in 150 ml Thionylchlorid nach Zusatz von 0,05 ml N,N-Dimethylformamid eine Stunde unter Rückfluß gekocht. Dann wird im Vakuum bis zur Trockne eingedampft und bis zur Gewichtskonstanz getrocknet.
Ausbeute: 3,30 g (100 % d. Th.) grüner Feststoff

| Analyse (bezogen auf wasserfreie Substanz) | | | | | |
|---|---|---|---|---|---|
| C 59,06<br>C 59,17 | H 3,43<br>H 3,50 | Cl 20,11<br>Cl 20,39 | N 5,30<br>N 5,14 | O 12,10 | ber. |

c) 10,15,20-Tetrakis-[N,N',N", N"'-(6-hydroxy-2,2-dimethyl-1,3-dioxepan-5-yl)-3-aminocarbonylmethoxyphenyl]-porphyrin

2,97 (2,81 mmol) der unter Beispiel 13b hergestellten Verbindung werden in 150 ml wasserfreiem Dichlormethan gelöst. Man gibt 1,70 g (16,8 mmol) Triethylamin und 2,72 g (16,8 mmol) trans-6-Amino-2,2-dimethyl-1,3-dioxepan-5-ol zu und rührt 6 h unter Feuchtigkeitsausschluß bei 20°C. Anschließend wird eingedampft und über Kieselgel mit Dichlormethan/Methanol chromatographiert. die Produktfraktronen werden eingedampft und aus Isopropanol/tert.-Butylmethylether umkristallisiert.
Ausbeute: 2,83 g (68 % d. Th.) violette Kristalle

| Analyse (bezogen auf wasserfreie Substanz) | | | | |
|---|---|---|---|---|
| C 64,77<br>C 64,57 | H 6,11<br>H 6,30 | N 7,55<br>N 7,63 | O 21,57<br>O 21,57 | ber. |

d) Mangan(III)-{Tetrakis-[N,N',N",N"'-(6-hydroxy-2,2-dimethyl-1,3-dioxepan-5-yl)-3-aminocarbonylmethoxyphenyl]-prophyrin}-acetat

2,54 g (1,71 mmol) der unter Beispiel 13 c hergestellten Verbindung und 10 g Mangan(II)-acetat-Tetrahydrat werden in einem Gemisch aus 200 ml Pyridin und 50 ml Wasser ca. 3 h bei 80°C gerührt. Man dampft im Vakuum ein, nimmt den Rückstand in 300 ml Dichlormethan wässriger Kochsalzlösung auf, trocknet die organische Phase über wasserfreiem Magnesiumsulfat und filtriert. Das Filtrat wird eingedampft und der Rückstand aus wässrigem Ethanol umkristallisiert.
Ausbeute: 2,60 g (95 % d. Th.) dunkelgrüne Kristalle

| Analyse (bezogen auf wasserfreie Substanz) | | | | | |
|---|---|---|---|---|---|
| C 61,73<br>C 61,52 | H 5,75<br>H 5,81 | Mn 3,44<br>Mn 3,38 | N 7,02<br>N 6,84 | O 22,06 | ber. |

e) Mangan(III)-{Tetrakis-[N,N',N",N"'-[(1-RS, 2-SR)-2,3-dihydroxy-1-hydroxymethylpropyl]-3-amino-carbonylmethoxyphenyl]-porphyrin}-chlorid

2,11 g (1,32 mmol) der unter Beispiel 13 d hergestellten Verbindung werden in 100 ml Methanol gelöst. Man tropft 100 ml zweinormale Salzsäure zu und rührt 3 h bei 20°C. Nach Eindampfen im Vakuum wird das Rohprodukt in Methanol gelöst und durch Zugabe von tert.-Butylmethylether gefällt.
Ausbeute: 1,81 g (97 % d. Th.) dunkelgrüne Kristalle

| Analyse (bezogen auf wasserfreie Substanz) | | | | | | |
|---|---|---|---|---|---|---|
| C 57,85<br>C 57,632 | H 5,14<br>H 5,27 | Cl 2,51<br>Cl 2,68 | Mn 3,89<br>Mn 3,72 | N 7,94<br>N 7,84 | O 22,67 | ber. |

**Beispiel 14**

Herstellung eines Kontrastmittels für die nuklearmedizinische Anwendung:
$^{64}$Cu-bzw. $^{67}$Cu-Komplex von 5,10,15,20-Tetrakis-[3-(carboxymethoxy)-phenyl]-porphyrin

1,0 ml einer salzsauren Lösung von 1,0 mCi $^{64}CuCl_2$ bzw. $^{67}CuCl_2$ werden mit gesättigter wässriger Natriumhydrogencarbonatlösung auf pH 7,5 eingestellt. Man gibt 2,0 mg (2,2 mmol) der unter Beispiel 13 a) hergestellten Verbindung zu und autoklaviert die Suspension eine Stunde bei 120°C. Nach Filtration über einen Membranfilter erhält man ein rötliches Filtrat das praktisch 100 % der eingesetzten Aktivität enthält.
Die Lösung ist gebrauchsfertig.

**Beispiel 15**

Herstellung eines Kontrastmittels für die nuklearmedizinische Anwendung:
$^{111}$In-Komplex von 5,10,15,20-Tetrakis-[3-(carboxymethoxy)-phenyl]-porphyrin

1,0 ml einer salzsauren Lösung von 1,0 mCi $^{111}InCl_3$ werden mit gesättigter, wässriger Natriumhydrogencarbonatlösung auf pH 7,5 eingestellt. Man gibt 2,0 mg (2,2 mmol) der unter Beispiel 13 a) hergestellten Verbindung zu und autoklaviert die Suspension eine Stunde bei 120°C. Nach Filtration über einen Membranfilter erhält man ein rötliches Filtrat, das praktisch 100 % der eingesetzten Aktivität enthält.
Die Lösung ist gebrauchsfertig.

**Beispiel 16**

Herstellung eines Kontrastmittels für die nuklearmedizinische Anwendung:
$^{99m}$Tc-Komplex von 5,10,15,20-Tetrakis-[3-(carboxymethoxy)-phenyl]-porphyrin

Zu 1,0 ml einer Lösung von 1,8 mCi 99mTechnetium(III)-Acetylaccetat in DMSO gibt man 2,2 mg (2,0 mmol) der unter Beispiel 13 a) hergestellten Verbindung und autoklaviert die Lösung mehrere Stunden bei 130°C. Anschließend wird gelöstes Porphyrin durch Zugabe von 1,0 ml tert.-Butylmethylether gefällt, über ein Membranfilter abgesaugt und in 1,0 ml pyrogenfreier 0,1 molarer wässriger Natriumhydrogencarbonatlösung aufgenommen. Nach Membranfiltration erhält man die gebrauchsfertige Lösung.

**Beispiel 17**

Bestimmung von Verträglichkeit und Relaxivity der erfindungsgemäßen Verbindungen

Die Verträglichkeit wurde orientierend an Mäusen (NMRI, Schering SPF, weibl., ca. 25 g) untersucht. Es wurden jeweils 0,5 mmol/kg der Substanz einer Maus intravenös appliziert. Verstarb das Tier, wurde die Dosis halbiert und entsprechend verfahren, ansonsten wurde 2 weiteren Mäusen die entsprechende Dosis appliziert. Der Beobachtungszeitraum betrug 7 Tage. Die Verträglichkeit wird mit > angegeben, wenn alle Tiere die entsprechende Dosis vertrugen, mit < wenn alle verstarben.

Zur Bestimmung der Relaxivity wurden die Relaxationszeiten der Substanzen in 4 verschiedenen Konzentrationen (0 - 1 mmol/l; gelöst in Aqua dest.; pH 7,4) am Relaxometer (minispec pc120; 20 MHz; 40°C) gemessen. Die Berechnung erfolgte aus:

$$\Delta\ (1/T_{1,2}\text{-}1/T_{1,2}\ \text{Leerwert})\ /\ \Delta\ \text{konz.} = R_{1,2}\ \text{(lineare Regression)}.$$

**Ergebnisse:**

Aufgrund der besseren Verträglichkeit und der deutlich höheren Relaxivity weisen die erfindungsgemäßen Verbindungen einen deutlich höheren Sicherheitsabstand auf als es dem Stand der Technik ( z.B. EP 0 336 879 A 1) entspricht.

| Verbindung nach ... | Verträglichkeit mmol/kg | Relaxivity R1 l/mmol/s | Produkt Verträglichkeit und Relaxivity |
|---|---|---|---|
| Beispiel 2 c | > 0,5 | 13 | > 6,5 |
| Beispiel 4c | ≥ 0,5 | 11,6 | > 5,8 |
| Beispiel 2 aus EP 0336 879 A1* | < 0,5 | 8,6 | < 4,3 |
| Beispiel 4 aus EP 0336 879 A1** | < 0,25 | 8,8 | < 2,2 |
| Beispiel 17 aus EP 0336 879 A1*** | < 0,5 | 9,2 | < 4,6 |

* Mangan(III)- {5,10,15,20-tetrakis-{[4-carbonsäure-(2,3-dihydroxy-1-hydroxymethylpropyl)-amid]-phenyl}-porphyrin}-acetat
** Mangan(III)-[5,10,15,20-tetrakis-(3-carboxylatophenyl)-porphyrin]-acetat
*** Mangan(III)-[5,10,15,20-tetrakis-(4-methoxy-3-sulfonatophenyl)-porphyrin]-acetat, Tetranatriumsalz

**Beispiel 18**

MR-Imagingexperiment von tu mortragenden (Prostatacarcinom) Nacktmäusen.

**Durchführung:**

**Aufnahmeparameter:** Bruker Biospec (2,35 Tesla; 100.33 MHz), Kernspintomographie, Klinikum Steglitz. Schichtorientierung axial Schichtdicke: 4 mm, Number of Averages: 4/slice, Field of View: 80 mm, Matrix: $256^2$, 3-6 Schichten 1 Echo pro Aufnahmesequenz, MultiSliceVariableEcho (MSVE): $T_R$: 400, $T_E$: 25

**Spezies:** Nacktmaus, NMRI, nu/nu, männl., = 25 g

**Tumormodell:** Prostatacarcinom (MAT/Lu), ca. 3 Wochen vor Versuchsbeginn subcutan implantiert, n= 2-3 (pro Substanz und Dosis).

**Dosierung:** 0,05 und 0,1 mmol/kg Körpergewicht i.v.

**Formulierung:** Erfindungsgemäße Verbindungen wurden gelöst in Tris-(hydroxymethyl)-aminomethan (50 mmol/l) NaCl - (100 mmol/l) - Puffer (Endkonzentration: 10 mmol/l; p H7,5)

**Ergebnisse:**

Die Applikation von 0,1 mmol/kg i.v. der erfindungsgemäßen Verbindungen führte (unabhängig von der Tumorgröße) zu einem guten, relativ homogenen Enhancement im Tumor, das im Verlauf der Untersuchungen (zumindest bis 120 min p.i.) noch anstieg. In Leber und Muskel war lediglich direkt nach Applikation ein in etwa vergleichbares Enhancement zu erkennen, das jedoch im Gegensatz zu dem im Tumor kontinuierlich abnahm, so daß sich der Tumor-Gewebe-Kontrast im Verlauf der Imagingversuche ständig verbesserte. Da die Substanz überwiegend renal eliminiert wird, stellten sich auch die Nieren schon direkt nach Applikation sehr hell dar. Allerdings nahm auch in den Nieren das Enhancement deutlich schneller ab als in den Tumoren, so daß auch hier ein verbesserter Tumor-Gewebe-Kontrast (ab ca. 60 min p.i.) festzustellen war.

Bei den Imagingversuchen mit 0,05 mmol/kg i.v. konnten insgesamt vergleichbare Ergebnisse erzielt werden (bei zwangläufig etwas geringerem Signalanstieg). In der Abbildung 1 ist diese Kinetik des Tumorenhancements bei zwei unterschiedlichen Dosierungen der Verbindung gemäß Beispiel 2d exemplarisch dargestellt. Insbesondere in der Dosierung 0.1 mmol/kg Körpergewicht führte die einmalige intravenöse Applikation der Verbindung über den gesamten Untersuchungs-zeitraum zu einer deutlich verbesserten Differenzierbarkeit zwischen Tumor und Normalgewebe.

Abbildung 1 zeigt ein $^1$H-NMR-Imaging von tumortragenden (Prostata-Carcinom) Nacktmäusen nach einmaliger intravenöser Applikation von Verbindung nach Beispiel 2d (Tier links (a), Dosis: 0,1 mmol/kg; Tier rechts (b), Dosis: 0,05 mmol/kg). Aufnahme vor Injektion (0 min), sowie 5 min, 60 min und 180 min p.i..

**Beispiel 19**

MR-Imaging-Experiment von tumortragenden (VX-2-Carcinom) Kaninchen.

**Durchführung:**

**Aufnahmeparameter:** Siemens Magnetom® (1,5 Tesla; 64 MHz), $T_1$-betonte Spin-Echo-Technik: $T_R$: 350 ms, $T_E$: 15 ms. Schichtorientierung: coronar, Schichtdicke: 3 mm, Number ofAverages: 4 pro Schicht, Field of View: 150 mm, Matrix: $256^2$, 4-6 Schichten 1 Echo pro Aufnahmesequenz.

**Spezies:** Hasen-Kaninchen, Wulf, weibl., = 4 kg Körpergewicht (KGW) [n=3].

**Tumormodell:** Carcinom (VX-2), ca. 3 Wochen vor Versuchsbeginn intramuskulär implantiert.

**Dosierung:** 0,05 und 0,1 mmol/kg Körpergewicht i.v.

**Ergebnisse:**

Die Applikation der Referenzsubstanz Magnevist® in der Dosis: 0.1 mmol/Kg i.v. führte nur zu einem kurzzeitigen geringen Signalanstieg im implantierten Tumor und in den auf der tumortragenden sowie auf der tumorfreien Seite untersuchten Lymphknoten (iliacales und popliteales). Innerhalb von rund 60-90 Minuten nach Applikation war in praktisch allen untersuchten Geweben wieder annähernd die Ausgangsintensität erreicht und nahezu kein Enhancement von Tumor bzw. Metastase mehr festzustellen.

Die Applikation von 0.05 mmol/kg KGW i. v. der erfindungsgemäßen Verbindungen führte zu einem guten langanhaltenden Enhancement und zwar sowohl im implantierten Tumor als auch in der Mehrzahl der Lymphknoten auf der tumortragenden Seite. Aufgrund des ausgeprägten Enhancements in diesen Lymphknoten bzw. Lymphknotenarealen, nach Applikation der erfindungsgemäßen Verbindungen, konnte daher ein Metastasen-Befall der Lymphknoten diagnostiziert werden. Die histologische Untersuchung der Lymphknoten bestätigte die Ergebnisse der MR-tomographischen Untersuchung.

| Gd-DTPA: 0,1 mmol/kg KGW i. v. | | | | |
|---|---|---|---|---|
| min p. i. | Lymphknoten (iliacales) | Lymphknoten-Metastase | VX-2-Carcinom | Muskel |
| 0 | 1,00 | 1,00 | 1,00 | 1,00 |
| 2 | 1,50 | 1,61 | 1,50 | 1,20 |
| 8 | 1,32 | 1,44 | 1,50 | 1,11 |
| 17 | 1,20 | 1,36 | 1,48 | 1,10 |
| 42 | 1,24 | 1,29 | 1,47 | 1,07 |
| 52 | 1,17 | 1,20 | 1,42 | 1,02 |
| 69 | 1,11 | 1,25 | 1,40 | 1,08 |
| 24 h p. i. | 1,14 | 0,97 | 1,11 | 1,04 |

Relative Signalintensität in verschiedenen Geweben eines tumortragenden (VX-2-Carcinom) Kaninchens (mit Metastase in einem iliakalen Lymphknoten) nach einmaliger intravenöser Applikation von Gd-DTPA (Magnevist®) (Dosis: 0.1 mmol/kg).
Die Ausgangssignalintensität (= SI $_{praekontrast}$) in den verschieden Geweben wurde jeweils gleich 1 gesetzt.

Verbindung gemäß Beispiel 2d): 0,05 mmol/kg KGW i. v. (bezogen auf Mol Porphyrin)

| min p. i. | Lymphknoten (iliacales) | Lymphknoten-Metastase | VX-2-Carcinom | Muskel |
|---|---|---|---|---|
| 0 | 1,00 | 1,00 | 1,00 | 1,00 |
| 2 | 1,80 | 1,68 | 1,28 | 1,14 |
| 28 | 1,93 | 1,45 | 2,19 | 1,17 |
| 44 | 2,00 | 2,15 | 1,70 | 1,19 |
| 61 | 1,91 | 2,18 | 1,48 | 1,21 |
| 76 | 1,84 | 2,32 | 1,45 | 1,19 |
| 94 | 1,65 | 2,12 | 1,44 | 1,13 |
| 111 | 1,58 | 2,21 | 1,50 | 1,18 |
| 24 h p. i. | 1,13 | 1,39 | 1,46 | 0,99 |

Relative Signalintensität in verschiedenen Geweben eines tumortragenden (VX-2-Carcinom) Kaninchens (mit Metastase in einem iliakalen Lymphknoten) nach einmaliger intravenöser Applikation der erfindungsgemäßen Verbindung (Dosis: 0.05 mmol/kg).
Die Ausgangssignalintensität (= SI $_{praekontrast}$) in den verschieden Geweben wurde jeweils gleich 1 gesetzt.

Die Abbildung 2 zeigt relative Signalintensität in verschiedenen Geweben eines tumortragenden (VX-2-Carcinom) Kaninchens nach einmaliger intravenöser Applikation von Magnevist$^{(R)}$ (Dosis: 0,1 mmol/kg).
Die Ausgangssignalintensität (= SI praekontrast) in den verschiedenen Geweben wurde jeweils gleich 1 gesetzt.

Die Abbildung 3 zeigt relative Signalintensität in verschiedenen Geweben eines tumortragenden (VX-2-Carcinom)

Kaninchens nach einmaliger intravenöser Applikation der erfindungsgemäßen Verbindung (Dosis: 0,05 mmol/kg). Die Ausgangssignalintensität (= SI praekontrast) in den verschiedenen Geweben wurde jeweils gleich 1 gesetzt.

**Patentansprüche**

1. Porphyrin-Komplexverbindungen bestehend aus einem meso-Tetraphenylporphyrinliganden der allgemeinen Formel I

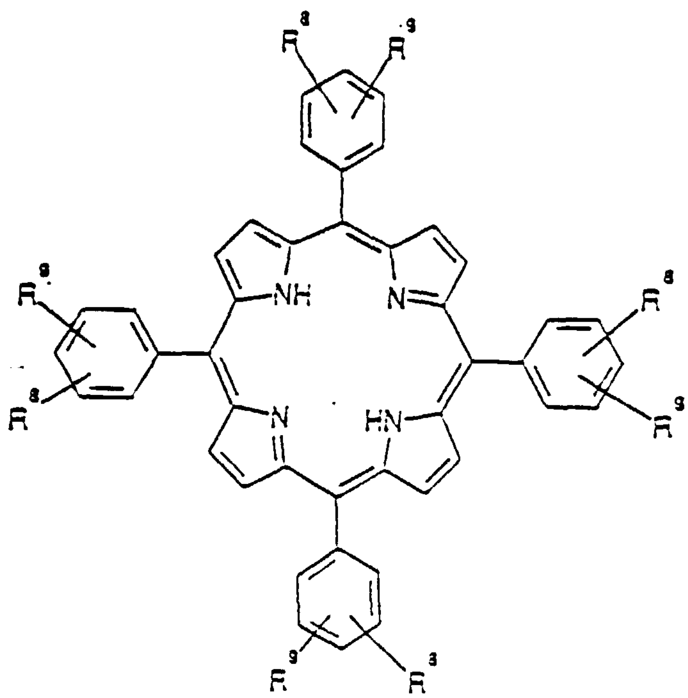

(I),

worin

$R^8$ für einen Rest $-(O)_s-(CH_2)_k-X-CH_2-Y-C(=O)-Z$ mit

s in der Bedeutung der Ziffern 0 oder 1,
k in der Bedeutung der Ziffern 0, 1, 2 oder 3,
X in der Bedeutung eines Sauerstoffatoms, einer direkten Bindung oder einer $-NR^{10}$-Gruppe wobei
$R^{10}$ für eine $C_1$-$C_4$-Acyl-, $C_1$-$C_{10}$-alkylsulfonyl-, Benzolsulfonyl-, $C_1$-$C_4$-alkylphenylensulfonyl-, Carboxy-$C_1$-$C_6$-alkyl oder Carboxy-$C_1$-$C_5$-acylgruppe,
Y in der Bedeutung einer direkten Bindung oder einer-CHOH-Gruppe und
Z in der Bedeutung einer-OH-oder $-N(R^{11})-R^{12}$ Gruppe steht, wobei
$R^{11}$ und $R^{12}$ unabhängig voneinander für Wasserstoff oder für einen gegebenenfalls durch 1 - 6 Hydroxygruppen substituierten gerad- oder verzweigtkettigen oder cyclischen, gesättigten oder ungesättigten Kohlenwasserstoffrest mit bis zu 16 C-Atomen stehen,
mit der Maßgabe, daß keine direkten O-O oder O-N-Bindungen gestattet sein sollen,

$R^9$ für ein Wasserstoff-, Fluor-, Chlor-, Brom-, Jodatom, einen geradkettigen oder verzweigten $C_1$-$C_4$-Alkyrest oder für einen der für $R^8$ angegebenen Substituenten stehen, und einem Ion eines Elements der Ordnungszahlen 21 - 32, 38, 39, 42 - 51 oder 58 - 83 sowie gegebenenfalls einem oder mehreren physiologisch unbedenklichen Kation(en) anorganischer und/oder organischer Basen, Aminosäuren oder Aminosäureamiden bestehen, mit Ausnahme der Eisen-, Nickel-, Mangan- und Zinn-Komplexe des meso-5,10,15,20-Tetrakis-[4-(carboxylatomethoxy)-phenyl]-porphyrins.

2. Verbindung nach Beispiel 2c:

Mangan(III)-{5,10,15,20-Tetrakis-[3-(carboxymethoxy)-phenyl]-porphyrin}-chlorid

Verbindung nach Beispiel 3d:
Mangan(III)-{5,10,15,20-Tetrakis-(2-(carboxymethoxy)-phenyl]-porphyrin}-chlorid, (4 Atropisomere)

Verbindung nach Beispiel 4 d:
Mangan(III)-{5,10,15,20-Tetrakis-[3,4-bis-(carboxymethoxy)-phenyl]-porphyrin}-chlorid

Verbindung nach Beispiel 5d:
Mangan(III)-{5,10,15,20-Tetrakis-[(N-acetyl-N-carboxymethyl)-aminophenyl]-3-porphyrin}-chlorid

Verbindung nach Beispiel 6c:
Mangan(III)-{5,10,15,20-Tetrakis-[3-(carboxymethyl)-phenyl]-porphyrin}-chlorid

Verbindung nach Beispiel 7c:
Mangan(III)-{5,10,15, 20-Tetrakis-[3-(2-carboxyethyl)-phenyl]-porphyrin}-chlorid

Verbindung nach Beispiel 8e:
Mangan(III)-{5,10,15,20-Tetrakis-{N,N-bis-(carboxymethyl)-3-aminophenyl-porphyrin}-chlorid

Verbindung nach Beispiel 9c:
Mangan(III)-{5,10,15,20-Tetrakis-[3-(2-carboxy-2-hydroxyethoxy)-phenyl]-porphyrin}-chlorid

Verbindung nach Beispiel 10c:
Mangan(III)-{5,-10,15,20-Tetrakis-[N-carboxymethyl)-N-(p-toluolsulfonyl)-3-aminomethylphenyl]-porphyrin}-chlorid

Verbindung nach Beispiel 11f:
Mangan(III)-{5,10,15,20-Tetrakis-[{3-(N-acetyl-N-carboxylmethyl-3-aminopropyloxy)-phenyl]-porphyrin}-chlorid

Verbindung nach Beispiel 12b:
Eisen (III)-{5,10,15,20-Tetrakis-[3-(carboxymethoxy)-phenyl]-porphyrin}-chlorid

Verbindung nach Beispiel 13e:
Mangan(III)-{Tetrakis-[N,N',N",N"'-[(1-RS,2-SR)-2,3-dihydroxy-1-hydroxymethylpropyl]-3-amino-carbonylmethoxyphenyl]-porphyrin}-chlorid

Verbindung nach Beispiel 14:
Kupfer-64- bzw. Kupfer-67-Komplex von 5,10,15,20-Tetrakis-[3-(carboxymethoxy)-phenyl]-porphyrin

Verbindung nach Beispiel 15:
Indium-111-Komplex von 5,10,15,20-Tetrakis-[3-(carboxymethoxy)-phenyl]-porphyrin

Verbindung nach Beispiel 16:
Technetium-99 m-Komplex von 5,10,15,20-Tetrakis-[3-(carboxymethoxy)-phenyl]-porphyrin

**3.** Pharmazeutische Mittel enthaltend mindestens eine Komplexverbindung gemäß Formel I von Anspruch 1, gegebenenfalls mit den in der Galenik üblichen Zusätzen.

**4.** Verwendung von mindestens einer Porphyrin-Komplexverbindung nach Anspruch 1 für die Herstellung von Mitteln für die NMR-Diagnostik, Radiodiagnostik oder Radiotherapie.

**5.** Verfahren zur Herstellung von Porphyrin-Komplexverbindungen bestehend aus einem meso-Tetraphenylporphyrinliganden der allgemeinen Formel I

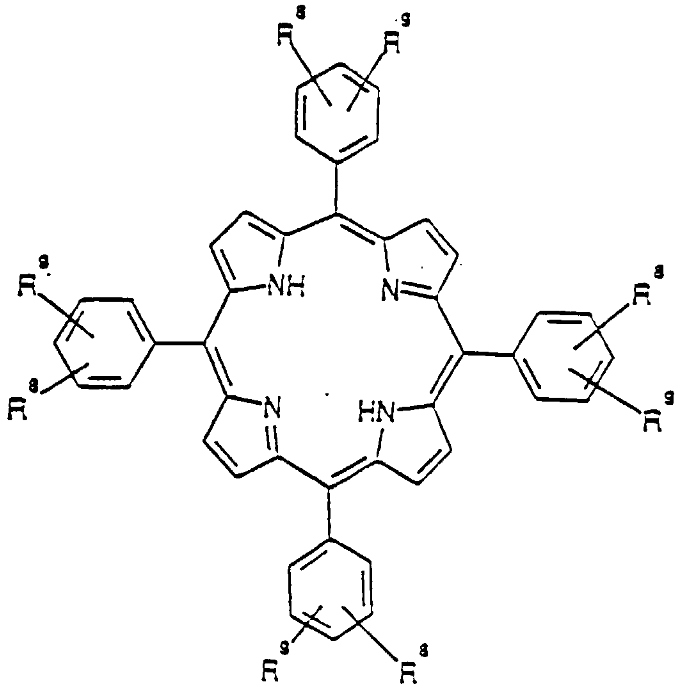

(I),

worin

$R^8$ für einen Rest $-(O)_s-(CH_2)_k-X-CH_2-Y-C(=O)-Z$ mit

s in der Bedeutung der Ziffern 0 oder 1,
k in der Bedeutung der Ziffern 0, 1, 2 oder 3,
X in der Bedeutung eines Sauerstoffatoms, einer direkten Bindung oder einer $-NR^{10}$-Gruppe wobei
$R^{10}$ für eine $C_1$-$C_4$-Acyl-, $C_1$-$C_{10}$-alkylsulfonyl-, Benzolsulfonyl-, $C_1$-$C_4$-alkylphenylensulfonyl-, Carboxy-$C_1$-$C_6$-alkyl oder Carboxy-$C_1$-$C_5$-acylgruppe,
Y in der Bedeutung einer direkten Bindung oder einer-CHOH-Gruppe und
Z in der Bedeutung einer-OH-oder $-N(R^{11})-R^{12}$ Gruppe steht, wobei
$R^{11}$ und $R^{12}$ unabhängig voneinander für Wasserstoff oder für einen gegebenenfalls durch 1 - 6 Hydroxygruppen substituierten gerad- oder verzweigtkettigen oder cyclischen, gesättigten oder ungesättigten Kohlenwasserstoffrest mit bis zu 16 C-Atomen stehen,
mit der Maßgabe, daß keine direkten O-O oder O-N-Bindungen gestattet sein sollen,

$R^9$ für ein Wasserstoff-, Fluor-, Chlor-, Brom-, Jodatom, einen geradkettigen oder verzweigten $C_1$-$C_4$-Alkyrest oder für einen der für $R^8$ angegebenen Substituenten stehen, und einem Ion eines Elements der Ordnungszahlen 21 - 32, 38, 39, 42 - 51 oder 58 - 83 sowie gegebenenfalls einem oder mehreren physiologisch unbedenklichen Kation(en) anorganischer und/oder organischer Basen, Aminosäuren oder Aminosäureamiden mit Ausnahme der Eisen-, Nickel-, Mangan- und Zinn-Komplexe des meso-5,10,15,20-Tetrakis-[4-carboxylatomethoxyl-phenyl]-porphyrins bestehen, dadurch gekennzeichnet, daß man Porphyrine der allgemeinen Formel II

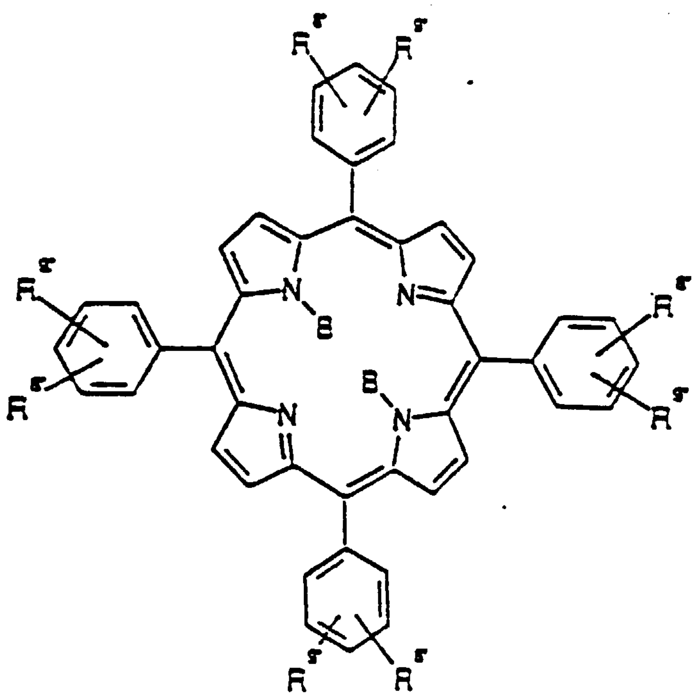

(II),

worin
B für ein Wasserstoffatom oder ein Metallionenäquivalent eines Elements der Ordnungszahlen 21-32, 38, 39, 42-51 oder 58-83,
$R^{8'}$ für einen Rest $-(O)_s-(CH_2)_k-X'-CH_2-Y'-C(=O)-Z'$ mit X' und Y' in der für X und Y angegebenen Bedeutung, wobei in X und Y gegebenenfalls enthaltene funktionelle Gruppen jedoch gegebenenfalls geschützt sind und Z' in der Bedeutung einer Fluchtgruppe,
$R^{9'}$ für $R^9$ oder für einen der für $R^{8'}$ angegebenen Substituenten stehen, verseift oder mit einem Amin der allgemeinen Formel III

$$H N R^{11'} R^{12'} \quad \text{(III)},$$

worin
$R^{11'}$ und $R^{12'}$ die für $R^{11}$ und $R^{12}$ angegebenen Bedeutung haben, wobei in $R^{11}$ und $R^{12}$ gegebenenfalls enthaltene Hydroxygruppen jedoch gegebenenfalls geschützt sind, umsetzt, gegebenenfalls vorhandene Schutzgruppen abspaltet und in den Fällen wo B für ein Wasserstoffatom steht, mit einem Metalloxid oder Metallsalz eines Elements der Ordnungszahlen 21-32, 38, 39, 42-51 oder 58-83 umsetzt - wobei die Reihenfolge der beiden letztgenannten Umsetzungen vertauscht werden kann - und gewünschtenfalls anschließend in den so erhaltendenen Porphyrinkomplexverbindungen gegebenenfalls noch vorhandene acide Wasserstoffatome durch Kationen anorganischer und/oder organischer Basen, Aminosäuren und Aminosäureamide substituiert.

**6.** Verfahren zur Herstellung der pharmazeutischen Mittel gemäß Anspruch 3, dadurch gekennzeichnet, daß man die in Wasser oder physiologischer Salzlösung gelöste oder suspendierte Komplexverbindung, gegebenenfalls mit den in der Galenik üblichen Zusätzen, in eine für die enterale oder parenterale Applikation geeignete Form bringt.

## Claims

**1.** Porphyrin complex compounds consisting of a meso-tetraphenylporphyrin ligand of the general formula I

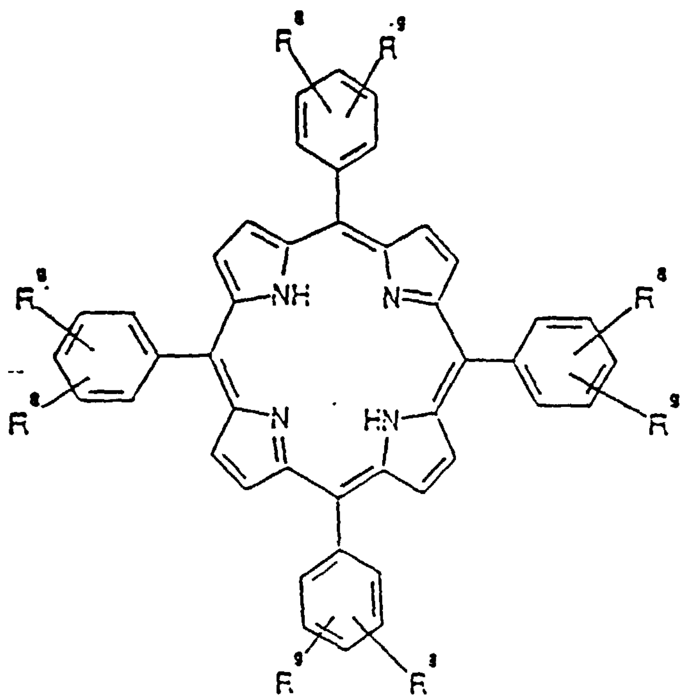

(I),

wherein

$R^8$ represents a radical $-(O)_s-(CH_2)_k-X-CH_2-Y-C(=O)-Z$ wherein

s denotes the number 0 or 1,
k denotes the number 0, 1, 2 or 3,
X denotes an oxygen atom, a direct bond or a $-NR^{10}$ group wherein
$R^{10}$ represents a $C_1$-$C_4$-acyl, $C_1$-$C_{10}$-alkylsulphonyl, benzenesulphonyl, $C_1$-$C_4$-alkylphenylenesulphonyl, carboxy-$C_1$-$C_6$-alkyl or carboxy-$C_1$-$C_5$-acyl group,
Y denotes a direct bond or a -CHOH group and
Z denotes an -OH or $-N(R^{11})-R^{12}$ group wherein
$R^{11}$ and $R^{12}$ represent independently of each other hydrogen or a straight-chain or branched or cyclic, saturated or unsaturated hydrocarbon radical having up to 16 carbon atoms that is optionally substituted by from 1 to 6 hydroxy groups,
with the proviso that no direct O-O or O-N bonds are to be permitted,

$R^9$ represents a hydrogen, fluorine, chlorine, bromine or iodine atom, a straight-chain or branched $C_1$-$C_4$-alkyl radical or one the substituents given for $R^8$,
and an ion of an element of atomic number 21 to 32, 38, 39, 42 to 51 or 58 to 83 and optionally one or more physiologically harmless cation(s) of inorganic and/or organic bases, amino acids or amino acid amides, with the exception of the iron, nickel, manganese and tin complexes of meso-5,10,15,20-tetrakis-[4-(carboxylatomethoxy)-phenyl]-porphyrin and meso-5,10,15,20-tetrakis-[4-(ethoxycarbonylmethoxy)-phenyl]-porphyrin.

**2.** Compound according to Example 2c:
manganese(III)-{5,10,15,20-tetrakis-[3-(carboxymethoxy)-phenyl]-porphyrin} chloride

Compound according to Example 3d:
manganese(III)-{5,10,15,20-tetrakis-[2-(carboxymethoxy)-phenyl]-porphyrin} chloride (4 atropisomers)

Compound according to Example 4d:
manganese(III)-{5,10,15,20-tetrakis-[3,4-(carboxymethoxy)-phenyl]-porphyrin} chloride

Compound according to Example 5d:
manganese(III)-{5,10,15,20-tetrakis-[(N-acetyl-N-carboxymethyl)-aminophenyl]-3-porphyrin} chloride

Compound according to Example 6c:
manganese(III)-{5,10,15,20-tetrakis-[3-(carboxymethyl)-phenyl]-porphyrin} chloride

Compound according to Example 7c:
manganese(III)-{5,10,15,20-tetrakis-[3-(2-carboxyethyl)-phenyl]-porphyrin} chloride

Compound according to Example 8e:
manganese(III)-{5,10,15,20-tetrakis-[N,N-bis-(carboxymethyl)-3-aminophenyl]-porphyrin} chloride

Compound according to Example 9c:
manganese(III)-{5,10,15,20-tetrakis-[3-(2-carboxy-2-hydroxyethoxy)-phenyl]-porphyrin} chloride

Compound according to Example 10c:
manganese(III)-{5,10,15,20-tetrakis-[N-carboxymethyl-N-(p-toluenesulphonyl)-3-aminomethylphenyl]-porphyrin} chloride

Compound according to Example 11f:
manganese(III)-{5,10,15,20-tetrakis-[3-(N-acetyl-N-carboxymethyl-3-amino-propyloxy)-phenyl]-porphyrin} chloride

Compound according to Example 12b:
iron(III)-{5,10,15,20-tetrakis-[3-(carboxymethoxy)-phenyl]-porphyrin} chloride

Compound according to Example 13e:
manganese(III)-{tetrakis-(N N',N",N"'-[(1-RS,2-SR)-2,3-dihydroxy-1-hydroxymethylpropyl]-3-amino-carbonylmethoxyphenyl]-porphyrin} chloride

Compound according to Example 14:
copper-64 or copper-67 complex of 5,10,15,20-tetrakis-[3-(carboxymethoxy)-phenyl]-porphyrin

Compound according to Example 15:
indium-111 complex of 5,10,15,20-tetrakis-[3-(carboxymethoxy)-phenyl]-porphyrin

Compound according to Example 16:
technetium-99 m complex of 5,10,15,20-tetrakis-[3-(carboxymethoxy)-phenyl]-porphyrin

**3.** Pharmaceutical agents containing at least one complex compound in accordance with formula I of claim 1, optionally with the additives customary in galenical pharmacy.

**4.** The use of at least one porphyrin complex compound according to claim 1 for the manufacture of agents for NMR diagnostics, radio diagnostics or radio therapy.

**5.** Process for the manufacture of prophyrin complex compounds consisting of a meso-tetraphenylporphyrin ligand of the general formula I

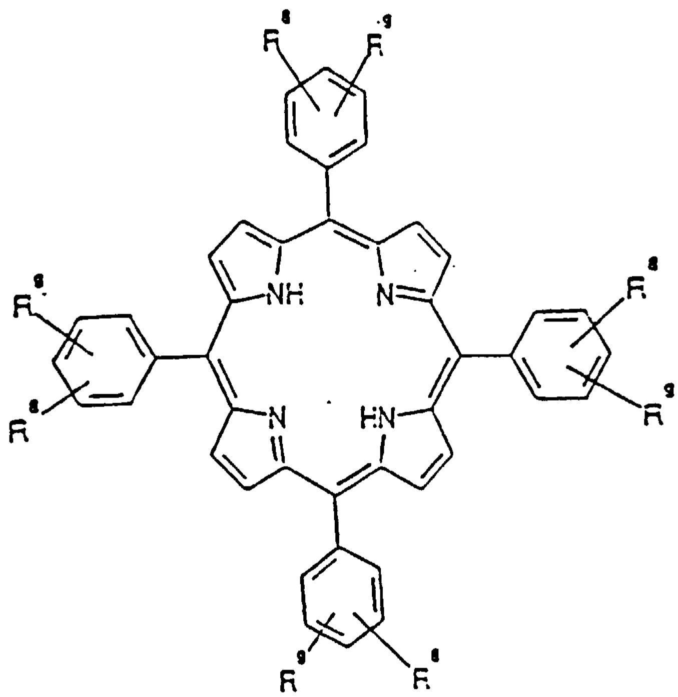

(I),

wherein

$R^8$ represents a radical $-(O)_s-(CH_2)_k-X-CH_2-Y-C(=O)-Z$ wherein

s denotes the number 0 or 1,
k denotes the number 0, 1, 2 or 3,
X denotes an oxygen atom, a direct bond or a $-NR^{10}$ group wherein
$R^{10}$ represents a $C_1$-$C_4$-acyl, $C_1$-$C_{10}$-alkylsulphonyl, benzenesulphonyl, $C_1$-$C_4$-alkylphenylenesulphonyl, carboxy-$C_1$-$C_6$-alkyl or carboxy-$C_1$-$C_5$-acyl group,
Y denotes a direct bond or a -CHOH group and
Z denotes an -OH or $-N(R^{11})-R^{12}$ group wherein
$R^{11}$ and $R^{12}$ represent independently of each other hydrogen or a straight-chain or branched or cyclic, saturated or unsaturated hydrocarbon radical having up to 16 carbon atoms that is optionally substituted by from 1 to 6 hydroxy groups,
with the proviso that no direct O-O or O-N bonds are to be permitted,
$R^9$ represents a hydrogen, fluorine, chlorine, bromine or iodine atom, a straight-chain or branched $C_1$-$C_4$-alkyl radical or one the substituents given for $R^8$,
and an ion of an element of atomic number 21 to 32, 38, 39, 42 to 51 or 58 to 83 and optionally one or more physiologically harmless cation(s) of inorganic and/or organic bases, amino acids or amino acid amides, characterised in that porphyrins of the general formula II

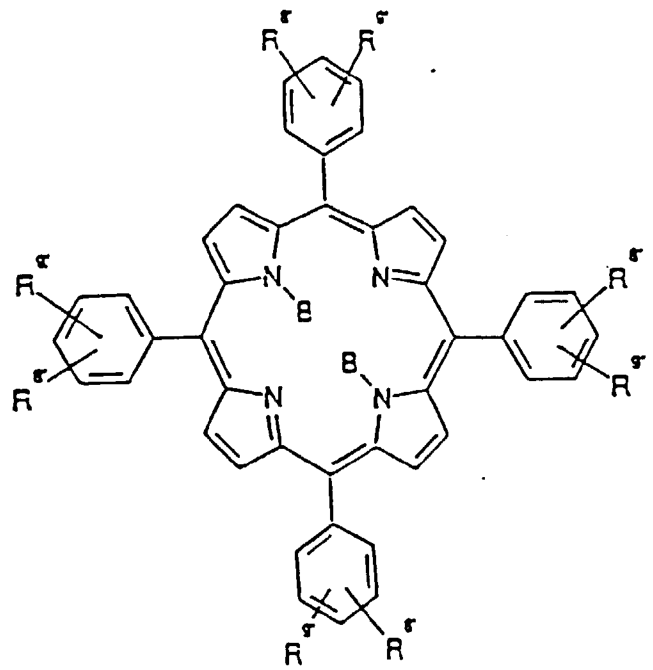

(II),

wherein
B represents a hydrogen atom or a metal ion equivalent of an element of atomic number 21 to 32, 38, 39, 42 to 51 or 58 to 83,
$R^{8'}$ represents a radical $-(O)_s-(CH2)_k-X'-CH2-Y'-C(=O)-Z'$ wherein X' and Y' having the meanings given for X and Y, functional groups optionally contained in X and Y, however, being optionally protected, and Z' represents a leaving group,
$R^{9'}$ represents $R^9$ or one of the substituents given for $R^{8'}$

are hydrolysed or are reacted with an amine of the general formula III

$$H N R^{11'} R^{12'} \qquad (III),$$

wherein
$R^{11'}$ and $R^{12'}$ have the meanings given for $R^{11}$ and $R^{12}$, hydroxy groups optionally contained in $R^{11}$ and $R^{12}$, however, being optionally protected, optionally present protecting groups are removed and, in cases where B represents a hydrogen atom, reacted with a metal oxide or metal salt of an element of atomic number 21 to 32, 38, 39, 42 to 51 or 58 to 83 - it being possible to reverse the order of the two last-mentioned reactions - and, if desired, acid hydrogen atoms optionally still present in the porphyrin complex compounds so obtained are subsequently substituted by cations of inorganic and/or organic bases, amino acids and amino acid amides.

6. Process for the manufacture of the pharmaceutical agents according to claim 3, characterised in that the complex compound, dissolved or suspended in water or physiological saline solution, is brought into a form suitable for enteral or parenteral administration, optionally with the additives customary in galenical pharmacy.

**Revendications**

1. Composés complexes de porphyrine composés d'un ligand de méso-tétraphénylporphyrine de formule générale I

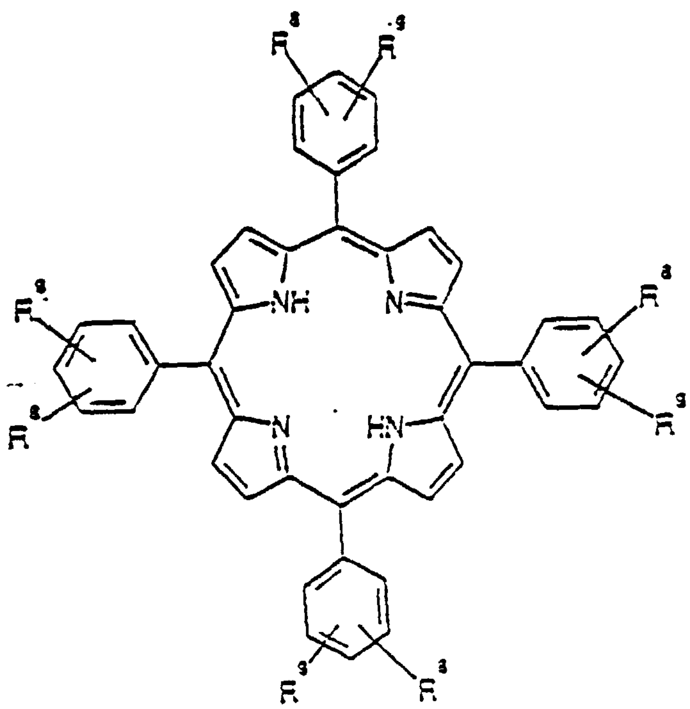

(I),

dans laquelle

$R^8$ représente un reste $-(O)_s-(CH_2)_k-X-CH_2-Y-C(=O)-Z$ avec

s vaut 0 ou 1,
k vaut 0, 1, 2 ou 3,
X représente un atome d'oxygène, une liaison directe ou un groupe $-NR^{10}$

$R^{10}$ représente un groupe acyle en $C_1$-$C_4$, (alkyl en $C_1$-$C_{10}$)sulfonyle, benzènesulfonyle, (alkyle en $C_1$-$C_4$)-Phénylsulfonyle, carboxy-(alkyle en $C_1$-$C_6$) ou carboxyacyle en $C_1$-$C_5$,

Y est une liaison directe ou un groupe -CHOH- et
Z représente un groupe -OH ou un groupe $-N(R^{11})-R^{12}$, où

$R^{11}$ et $R^{12}$, indépendamment l'un de l'autre, étant un atome d'hydrogène ou un reste hydrocarboné avec jusqu'à 16 atomes de carbone, linéaire ou ramifié ou cyclique, saturé ou insaturé, éventuellement substitué par 1 à 6 groupes hydroxyle,

à la condition qu'aucune liaison directe O-O ou O-N ne soit possible,
$R^9$ représente un atome d'hydrogène, de fluor, de chlore, de brome, d'iode, un reste alkyle en $C_1$-$C_4$ linéaire ou ramifié, ou un des substituants indiqués pour $R^8$ et un ion avec les numéros atomiques 21-32, 38, 39, 42-51 ou 58-83, ainsi qu'éventuellement un ou plusieurs cations physiologiquement inoffensifs de bases inorganiques et/ou organiques, d'acides aminés ou d'amides d'acides aminés, à l'exception des complexes de fer, de nickel, de manganèse et d'étain de la méso-5,10,15,20-tétrakis-[4-(carboxylatométhoxy)-phényl]porphyrine et de la méso-5,10,15,20-tétrakis-[4-(éthoxycarbonylméthoxy)-phényl]-porphyrine.

**2.** Composé selon l'Exemple 2c :
chlorure de manganèse (III)-{5,10,15,20-tétrakis-[3-(carboxyméthoxy)-phényl]-porphyrine}
Composé selon l'Exemple 3d :
chlorure de manganèse (III)-{5,10,15,20-tétrakis-(2-(carboxyméthoxy)-phényl]-porphyrine}, 4-atropisomères)
Composé selon l'Exemple 4d :
chlorure de manganèse (III)-{5,10,15,20-tétrakis-[3,4-bis-(carboxyméthoxy)-phényl]-porphyrine}
Composé selon l'Exemple 5d :
chlorure de manganèse (III)-{5,10,15,20-tétrakis-[(N-acétyl-N-carboxyméthyl)-aminophényl]-3-porphyrine}
Composé selon l'Exemple 6c :
chlorure de manganèse (III)-{5,10,15,20-tétrakis-[3-(carboxyméthyl)-phényl]-porphyrine}

Composé selon l'Exemple 7c :
chlorure de manganèse (III)-{5,10,15,20-tétrakis-[3-(2-carboxyéthyl)-phényl]-porphyrine}
Composé selon l'Exemple 8e :
chlorure de manganèse (III)-{5,10,15,20-tétrakis-{N,N-bis(carboxyméthyl)-3-aminophényl-porphyrine}
Composé selon l'Exemple 9c :
chlorure de manganèse (III)-{5,10,15,20-tétrakis-[3-(2-carboxy-2-hydroxyéthoxy)-phényl]-porphyrine}
Composé selon l'Exemple 10c :
chlorure de manganèse (III)-{5,10,15,20-tétrakis-[N-carboxyméthyl)-N-(p-toluène-sulfonyl)-3-aminométhylphényl]-porphyrine}
Composé selon l'Exemple 11f :
chlorure de manganèse (III)-{5,10,15,20-tétrakis-{3-(N-acétyl-N-carboxyméthyl-3-aminopropyloxy)-phényl]-porphyrine}
Composé selon l'Exemple 12b :
chlorure de fer (III)-{5,10,15,20-tétrakis-{N,N-bis-[3-(carboxyméthoxy)-phényl]-porphyrine}
Composé selon l'Exemple 13e :
chlorure de manganèse (III)-{tétrakis-[N,N',N',N "'- [(1-RS-2-SR)-2,3-dihydroxy-1-hydroxyméthyl-propyl]-3-amino-carbonylméthoxyphényl]-porphyrine}
Composé selon l'Exemple 14 :
Complexe de $^{64}$cuivre respectivement de $^{67}$cuivre de la 5,10,15,20-tétrakis-[3-(carboxyméthoxy)-phényl]-porphyrine.
Composé selon l'Exemple 15 :
Complexe de $^{111}$indium de la 5,10,15,20-tétrakis-[3-(carboxy-méthoxy)-phényl]porphyrine.
Composé selon l'Exemple 16 :
Complexe de $^{99m}$technétium de la 5,10,15,20-tétrakis-[3-(carboxyméthoxy)-phényl]porphyrine.

**3.** Produit pharmaceutique contenant au moins un composé complexe de formule I de la revendication 1, éventuellement avec des additifs usuels en galénique.

**4.** Utilisation d'au moins un composé complexe de porphyrine selon la revendication 1, pour la préparation de produits pour le diagnostic par la RMN, le radiodiagnostic ou la radiothérapie.

**5.** Procédé pour la préparation de composés complexes de porphyrine, composés d'un ligand méso-tétraphénylporphyrine de formule générale I

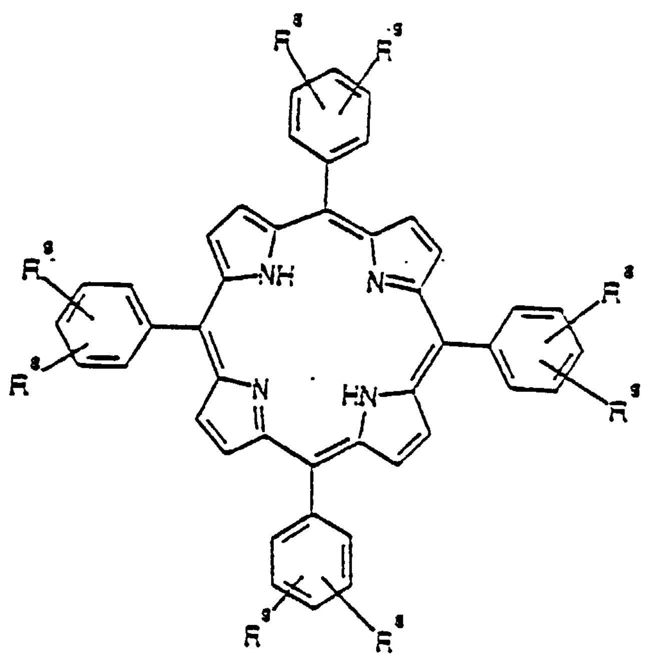

(I),

dans laquelle

$R^8$ représente un reste $-(O)_s-(CH_2)_k-X-CH_2-Y-C(=O)-Z$ avec

s vaut 0 ou 1,
k vaut 0, 1, 2 ou 3,
X représente un atome d'oxygène, une liaison directe ou un groupe $-NR^{10}$
$R^{10}$ représente un groupe acyle en $C_1$-$C_4$, (alkyl en $C_1$-$C_{10}$)-sulfonyle, benzènesulfonyle, (alkyl en $C_1$-$C_4$)-phénylsulfonyle, carboxy-(alkyle en $C_1$-$C_6$) ou carboxyacyle en $C_1$-$C_5$,
Y est une liaison directe ou un groupe -CHOH- et
Z représente un groupe -OH ou un groupe $-N(R^{11})-R^{12}$, où
$R^{11}$ et $R^{12}$, indépendamment l'un de l'autre, étant un atome d'hydrogène ou un reste hydrocarboné avec jusqu'à 16 atomes de carbone, linéaire ou ramifié ou cyclique, saturé ou insaturé, éventuellement substitué par 1 à 6 groupes hydroxyle,
à la condition qu'aucune liaison directe O-O ou O-N ne soit possible,

$R^9$ représente un atome d'hydrogène, de fluor, de chlore, de brome, d'iode, un reste alkyle en $C_1$-$C_4$ linéaire ou ramifié, ou un des substituants indiqués pour $R^8$ et un ion avec un numéro atomique 21-32, 38, 39, 42-51 ou 58-83, ainsi qu'éventuellement un ou plusieurs cations physiologiquement inoffensifs de bases inorganiques et/ou organiques, d'acides aminés ou d'amides d'acides aminés, caractérisé en ce que l'on saponifie des porphyrines de formule générale II

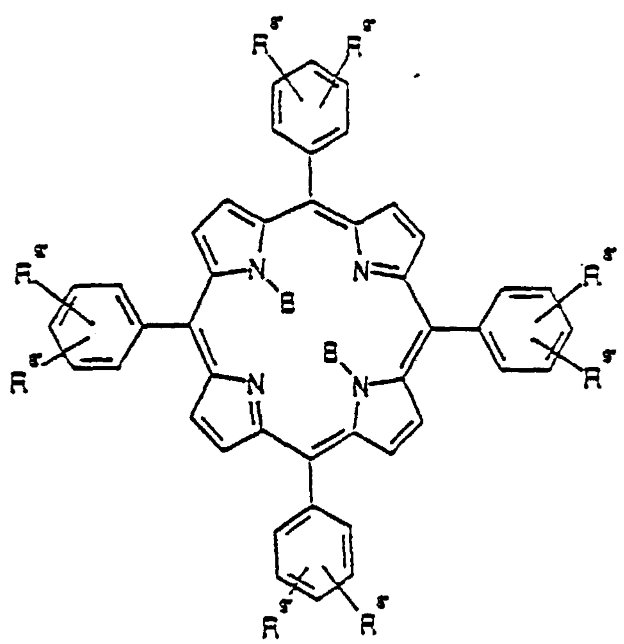

(II),

dans laquelle
B représente un atome d'hydrogène ou un équivalent d'ion métallique d'un élément avec un numéro atomique 21-32, 38, 39, 42-51 ou 58-83,

$R^{8'}$ représente un reste $-(O)_s-(CH_2)_k-X'-CH_2-Y'-C(=O)-Z'$ avec X' et Y' ayant les significations données pour X et Y, toutefois les groupes fonctionnels éventuellement contenus dans X et Y étant éventuellement protégés et Z' ayant la signification d'un groupe partant,

$R^{9'}$ représente $R^9$ ou un substituant donné pour $R^{8'}$,
ou en faisant réagir avec une amine de formule générale III

$$H\,N\,R^{11'}\,R^{12'} \qquad (III)$$

dans laquelle
$R^{11'}$ et $R^{12'}$ ont les significations données pour $R^{11}$ et $R^{12}$, les groupes hydroxy éventuellement contenus dans $R^{11}$ et $R^{12}$ étant toutefois éventuellement protégés, en dissociant les groupes protecteurs éventuellement présents et dans les cas où B représente un atome d'hydrogène, en faisant réagir avec un oxyde métallique ou un sel métallique d'un élément avec le numéro atomique 21-32, 38, 39, 42-51 ou 58-83 - la succession des deux dernières réactions citées pouvant être inversée - et si on le souhaite, en substituant les atomes d'hydrogène acides éventuellement encore présents dans les complexes de porphyrine ainsi obtenus, en utilisant des cations des bases inorganiques et/ou organiques, des acides aminés et des amides d'acides aminés.

**6.** Procédé pour la préparation de produits pharmaceutiques selon la revendication 3, caractérisé en ce qu'on met en forme pour des applications entérales ou parentérales le composé complexe dissous ou mis en suspension dans l'eau ou dans une solution physiologique de chlorure de sodium, éventuellement avec des adjuvants usuels en galénique.

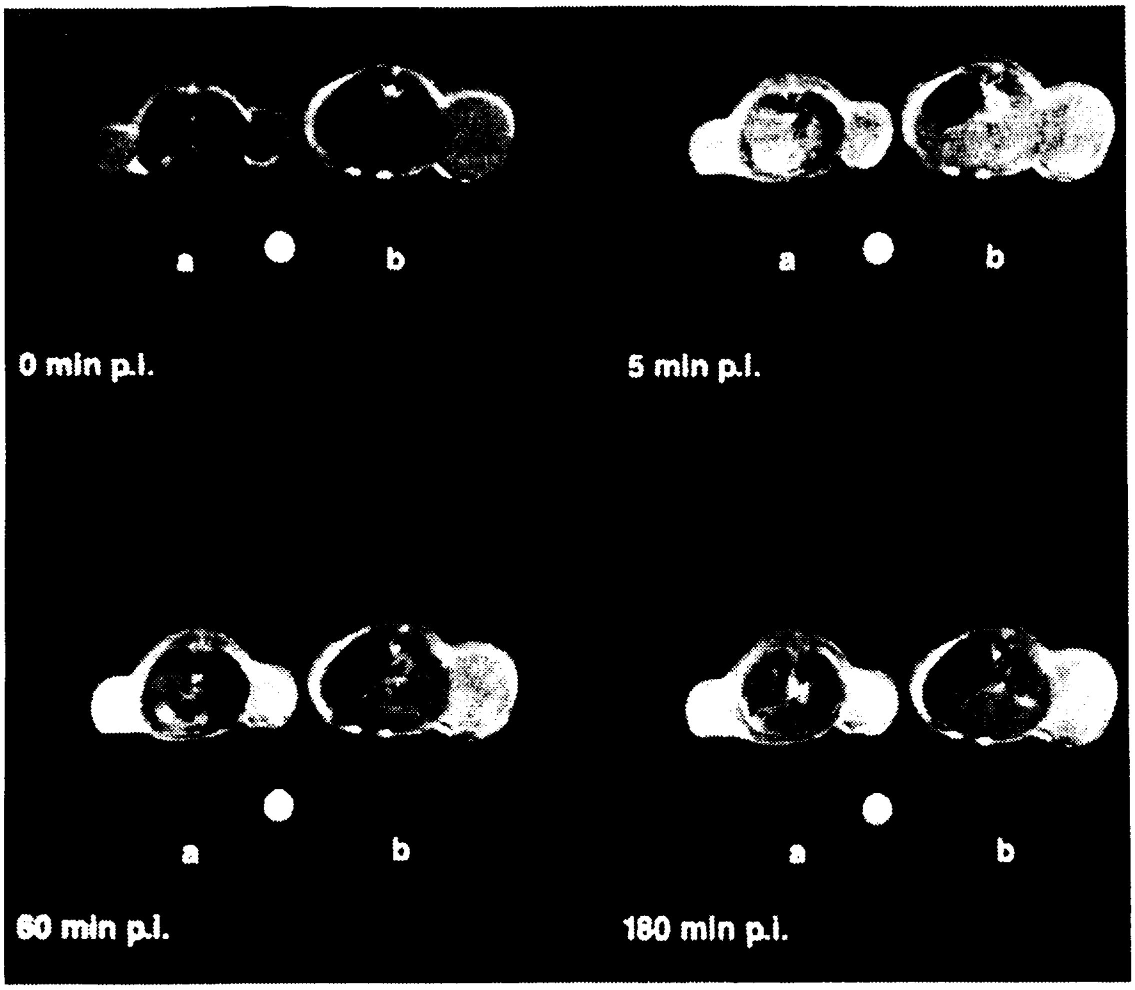


Abb. 1

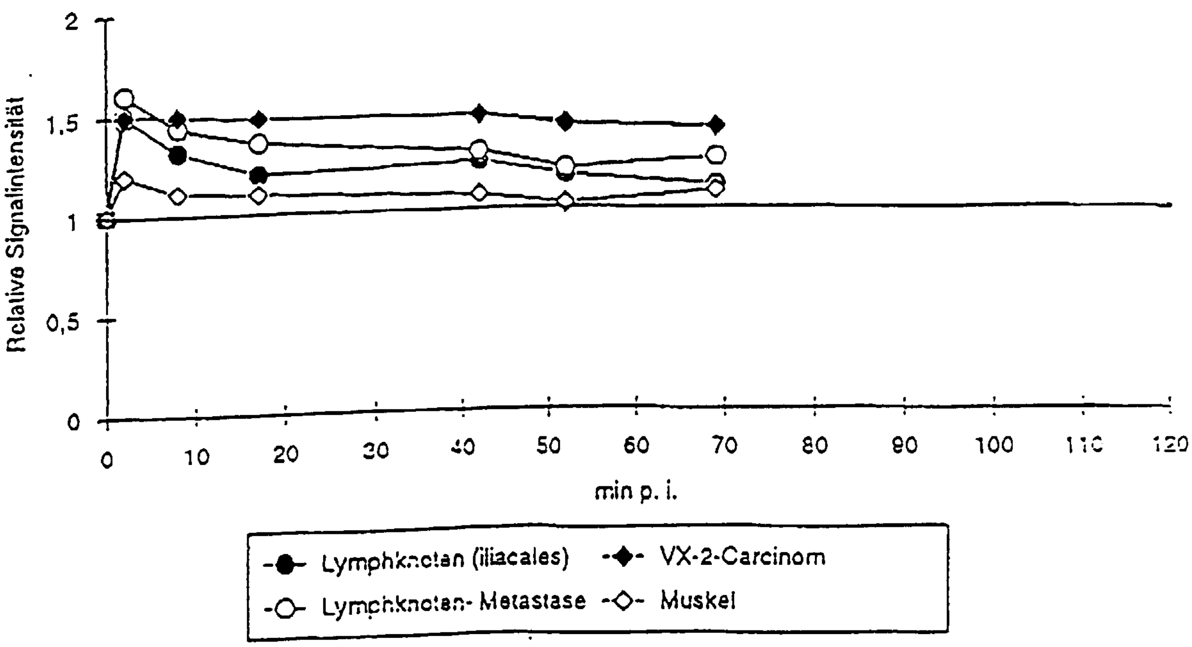

2
1,5
1
0,5
0
Relative Signalintensität
0
10
20
30
40
50
60
70
80
90
100
110
120
min p. i.
Lymphknoten (iliacales)
VX-2-Carcinom
Lymphknoten- Metastase
Muskel


Abb. 2

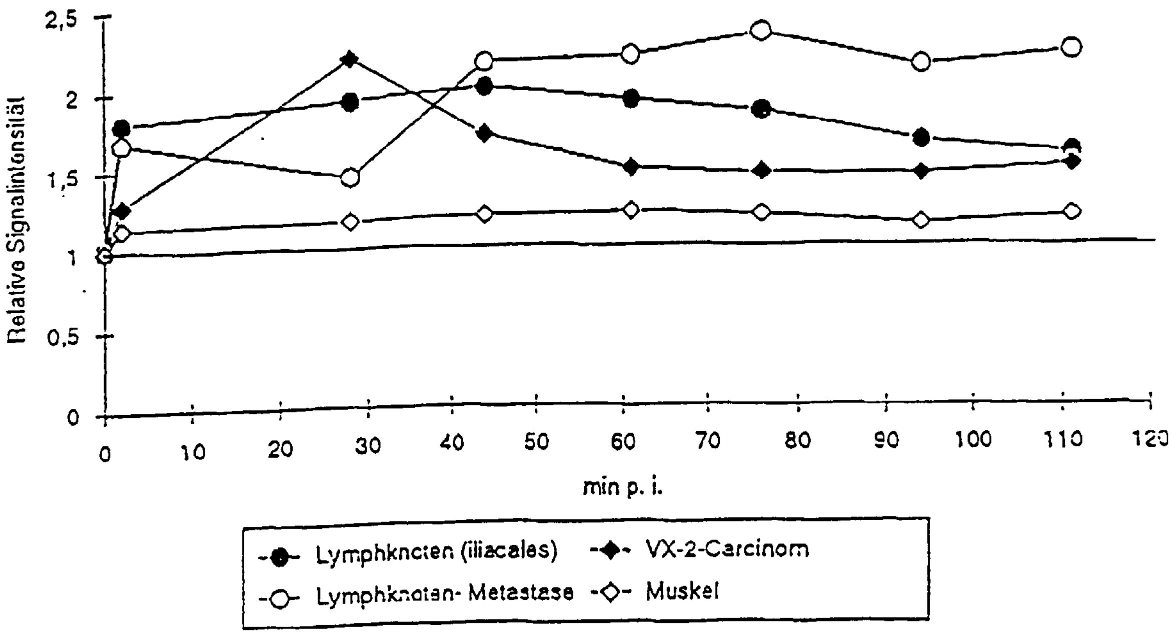

2,5
2
1,5
1
0,5
0
Relative Signalintensität
0
10
20
30
40
50
60
70
80
90
100
110
120
min p. i.
Lymphknoten (iliacales)
VX-2-Carcinom
Lymphknoten- Metastase
Muskel


Abb. 3